# EUROPEAN PATENT APPLICATION

(11) **EP 4 023 744 A1**
(43) Date of publication of application: **06.07.2022**
(21) Application number: 20397519.8
(22) Date of filing: 31.12.2020
(51) Int. Cl.: C12M 1/12, C12M 1/00, C12M 1/26, C12N 5/00

(54) **A BIOREACTOR AND A METHOD FOR SEPARATING CELL-DERIVED PRODUCTS FROM CULTURED CELLS AND A NANOSTRUCTURED CELLULOSE PRODUCT**

(71) Applicant: UPM-Kymmene Corporation, 00100 Helsinki (FI)
(72) Inventor: Nuopponen, Markus, 00220 Helsinki (FI); Sheard, Jonathan, Calcot, Reading, Berkshire RG31 7PQ (GB); Kiuru, Tony, 00440 Helsinki (FI); Paasonen, Lauri, 04440 Järvenpää (FI); Ståhlberg, Roosa, 33900 Tampere (FI); Mikkonen, Piia, 00250 Helsinki (FI); Meriluoto, Anne, 00100 Helsinki (FI)
(74) Representative: Berggren Oy

(57) **Abstract**

The present disclosure provides a bioreactor for extracting cell-derived products from cultured cells, the bioreactor comprising a container, an inlet for inputting cell culture medium into the container, an outlet for outputting cell culture medium comprising cell-derived products from the container, the container comprising, or being connected to, a compartment comprising nanostructured cellulose configured to receive cells, said compartment comprising a first separating surface separating the nanostructured cellulose from the outlet and allowing cell culture medium comprising cell-derived products to pass through the first separating surface. The present disclosure also provides a method for separating cell-derived products from cultured cells and a nanostructured cellulose product.

## Description

### Technical field

The present application relates to a bioreactor and to a method for separating cell-derived products from cultured cells. More particularly the present application relates to bioreactors and methods utilizing nanostructured cellulose as cell culture matrix.

### Background

Living cells release vesicles into local environment, which are called extracellular vesicles (EV). In general there are three main subclasses of EVs: microvesicles, apoptic bodies and exosomes. Microvesicles are shed from cell membrane directly and they have a diameter in the range of 50-1000 nm. Apoptotic bodies are derived from dying cells and have a diameter in the range of 50-4000 nm. Exosomes are released from multivesicular bodies (MVBs) rather than from cell membranes and have diameter in the range of 20-150 nm. Exosomes may contain RNA, DNA and protein molecules. Exosomes are communication vehicles that transfer bioactive proteins and generic material between cells.

EVs can act as mediators of cell signalling since they are able to transfer RNA and protein instructional cues and this could potentially be used for medical purposes. There is also evidence that EVs might stimulate regeneration or modulate pathological conditions and thus EVs could be used as new potential therapeutic agents in therapeutic methods. EVs may also be used a diagnostic biomarkers of disease such as cancer, for example based on unique miRNA profiles and other carbo that is associated with a pathological effect. They may also be used as biomarkers of infectious disease, based on that fact that they transmit infection-specific elements. Furthermore, EVs could be exploited as targeted drug delivery vehicles for the treatment of cancer, for example.

However, no practical manufacturing process exist to generate clinically relevant quantities of EVs. Unless efficient and cost-effective production methods can be obtained, it is difficult to implement real-life therapeutic uses for the artificially produced EVs. Especially it is desired to scale up cell culture methods and produce exosomes as large-scale stem cell cultures. Currently these are rate-limiting steps for delivering products for therapeutic applications.

### Summary

It was found out how cell-derived products could be efficiently produced in a cell culture in a bioreactor using nanostructured cellulose, such as nanofibrillar cellulose, as matrix for the cell culture, and how the cell-derived products could be separated from the cells and thereby harvested.

The present application provides a bioreactor 10 for extracting cell-derived products from cultured cells, the bioreactor comprising
- a container 12,
- an inlet 14 for inputting cell culture medium into the container,
- an outlet 16 for outputting cell culture medium comprising cell-derived products from the container,
- the container 12 comprising, or being connected to, a compartment 18 comprising nanostructured cellulose configured to receive cells, said compartment comprising a first separating surface 20 separating the nanostructured cellulose from the outlet and allowing cell culture medium comprising cell-derived products to pass through the first separating surface 20.

The present application also provides a method for extracting cell-derived products from cultured cells, the method comprising
- providing the bioreactor,
- providing cell culture medium, preferably serum-free, animal origin free, feeder-free and/or xeno-free cell culture medium,
- providing cells,
- incubating the cells in the compartment comprising nanostructured cellulose to form cell-derived products, and optionally mixing the nanostructured cellulose and the cells,
- allowing cell-derived products to diffuse from the cells into the cell culture medium,
- harvesting the cell culture medium comprising the cell-derived products from the bioreactor, preferably via the outlet for extracting the cell culture medium comprising the cell-derived products, to separate the cell-derived products from the incubated cells.

The present application provides a nanostructured cellulose product for extracting cell-derived products from cultured cells, the product comprising chemically unmodified nanostructured cellulose hydrogel having a median pore size in the range of 0.1-10 µm², such as 0.3-2.0 µm².

The main embodiments are characterized in the independent claims. Various embodiments are disclosed in the dependent claims. The embodiments and examples disclosed herein are mutually freely combinable unless otherwise explicitly stated.

The nanostructured cellulose may be nanofibrillar cellulose or nanocrystalline cellulose. These different materials enable providing products, bioreactors and methods with different and/or adjustable properties and suitable for different uses.

Nanofibrillar cellulose or nanocrystalline cellulose, when present as a hydrogel, are able to form a hydrophilic interstitial matrix for cells, which matrix is non-toxic, biocompatible and also biodegradable. The matrix can be degraded enzymatically, for example by adding cellulase. On the other hand the hydrogel is stable at physiological conditions, and does not need to be crosslinked by using additional agents. The properties, such as porosity and permeability, of the hydrogel may be controlled by adjusting the chemical and/or physical properties of the nanostructured cellulose.

Certain advantageous properties of the hydrogel comprising nanostructured cellulose, such as nanofibrillar cellulose, include flexibility, elasticity and remouldability, which enable for example using a variety of bioreactors for culturing and handling the material, such as conveying through inlets or outlets, and mixing. The hydrogel has optimal elasticity, stiffness, shear stress, mechanical adhesion and porosity to be also used as 3D and 2D cell storage or culture matrix. The cells may adhere or be embedded inside the matrix of the hydrogel used. The hydrogel also protects the cells during the production.

As the hydrogel contains a lot of water, it also shows good permeability for molecules. The hydrogels of the embodiments also provide high water retention capacity and molecule diffusion property speed.

The nanostructured cellulose hydrogels described herein are useful in medical and scientific applications, wherein the materials comprising nanostructured cellulose are in contact with living matter. The products containing nanostructured cellulose as described herein are highly biocompatible with the biological matter and provide several advantageous effects. Without binding to any specific theory, it is believed that a hydrogel comprising very hydrophilic nanostructured cellulose having a very high specific surface area, and thus high water retention ability, when applied against cells, provides favourable moist environment between the cells and the hydrogel comprising nanostructured cellulose. The high amount of free hydroxyl groups in the nanostructured cellulose forms hydrogen bonds between the nanostructured cellulose and water molecules and enables gel formation and the high water retention ability of the nanostructured cellulose. The nanostructured cellulose hydrogel contains a high amount of water, and it also enables migration of fluids and/or substances, especially bioactive substances in active form. It was surprisingly found out that despite the high amount of free hydroxyl groups in the nanostructured cellulose the material did not interact harmfully with the biologics produced by the cells thus allowing the biologics to be released from the nanostructured cellulose for extraction.

The nanostructured cellulose can be used as a matrix for cells in the production of cell-derived products thus providing an environment, which protects the cells and helps them to maintain their viability. The formed matrix, which may be called interstitial matrix, physically resembles ECM and provides a meshwork-like matrix which can be provided with desired pore sizes. The dimensions of the network of nanostructured cellulose, especially cellulose nanofibrils, is very close to natural ECM network of collagen nanofibrils. It provides structural support for cells and a network of interconnected pores for efficient cell migration and transfer of nutrients to the cells and cell-derived products from the cells. The nanostructured cellulose can stand the flow used in a continuous bioreactor system and the cells are maintained in the nanostructured cellulose matrix, such as maintained at their locations and maintained at a desired state, for example at a desired state of undifferentiation in case of stem cells. This enables obtaining the same cell-derived product(s) during the whole production time.

Furthermore, nanostructured cellulose is non-animal-based material and therefore xeno-free, so there is no risk for disease transfer or rejection. Especially when human cells are concerned, the formed system can comprise, in addition to the cellulose and probably minor amount of additives, only human-derived components, so it does not contain any material from foreign animal or microbial species.

Cellulose nanofibrils and nanocrystals have negligible fluorescence background. With the present materials it is possible to obtain a transparent and porous matrix for the cells, and the handling of the material is easy compared to the alternatives.

Cellulose is biocompatible due to moderate, if any, foreign responses and is safe especially for stem-cell applications, with no known toxicity. It is also biodurable; cellulose resorption is slow, as cells cannot synthesize cellulases required to degrade cellulose.

The present solutions are especially suitable for stimulating *in vivo* like biologics secretion, such as EV secretion, and the exploration of the sustained production of the biologics. For example, many of the cells cultured for exosomes are anchorage-dependent, thus NFC hydrogel, beads or the like entities or forms, which can incorporate the cells are especially suitable for culturing exosome-secreting cells. This also applies to other types of cell-derived products as well, such as secreted or excreted biologics. In one solution a mixture of nanostructured cellulose and cells is maintained while cell-derived product enriched media is harvested at a rate equal to addition of new media. A permeabilized or semi-permeable structure such as a membrane or a filter can be used to separate nanostructured cellulose and cells from the cell-derived product enriched medium. The cell-derived products, such as extracellular vesicles, may be harvested by using any suitable methods, such as based on their sizes, for example by using size exclusion chromatography, and/or functional molecules, such as biomolecules binding an EV surface protein.

The present materials and arrangements enable culturing cells in a form suitable for facilitating efficient growth and cell culture production. The cells may be for example grown as aggregates, such as spheroids, which may be desired for cell culture production purposes. Spheroid cultures can be maintained for weeks, even months, in nanostructured cellulose hydrogel allowing efficient utilization of the cells in the bioreactor. The use of nanostructured cellulose as interstitial matrix may prevent or decrease undesired agglomeration of the cells or cell spheroids and help to maintain the desired phenotype of the cells and the spectrum of produced cell-derived products.

Also, the nanostructured cellulose shelters the cells as well as the cell-derived products, which is especially important in case the cell-derived products are vesicles or the like fragile structures, during the process reducing issues associated with hydrodynamic shear forces in bioreactors. The nanostructured cellulose may be provided in such form that facilitates flow of liquids and diffusions of substances, such as the cell culture products but also nutrients, metabolites, or other substances having an impact to the process.

The culture surface area can be maximized by using scaling-up technologies, such as microcarriers in stirred-tank reactors or culture in fixed-bed or hollow-fiber bioreactors. Two-compartment culture in flask is also possible.

### Brief description of the drawings

Figure 1 shows an example of a bioreactor comprising nanofibrillar cellulose in the compartment.
Figure 2 shows an example of a bioreactor comprising nanofibrillar cellulose in the compartment.
Figure 3 shows an example of a bioreactor comprising nanofibrillar cellulose in the compartment.
Figure 4 shows determined fibril diameter and pore size of native nanofibrillar cellulose measured at 0.5 wt%.
Figure 5 shows determined fibril diameter and pore size of anionic nanofibrillar cellulose measured at 0.5 wt%.
Figure 6 shows an example of a bioreactor implemented in a cell culture flask.
Figure 7 shows an example of a bioreactor comprising a separate fixed bed reactor for immobilizing the cells.

### Detailed description

In this specification, percentage values, unless specifically indicated otherwise, are based on weight (w/w). If any numerical ranges are provided, the ranges include also the upper and lower values. The open term "comprise" also includes a closed term "consisting of" as one option. The diameters disclosed herein, unless specifically indicated otherwise, refer to the smallest diameter, and may be defined as average or number-average diameter and may be determined microscopically.

The materials and products described herein may be medical and/or scientific materials and products, such as life science materials and products, and may be used in the methods and the applications involving living cells and/or bioactive material or substances, such as described herein.

The present methods and devices can be used to produce and/or separate, isolate and/or harvest cell-derived products. The cell-derived products are obtained from the cells, *i.e*. the cells provide or produce the cell-derived products. The cell-derived products may comprise vesicles, such as extracellular vesicles, for example exosomes, but they may also comprise other applicable cell-derived products, such as macromolecules, cell organelles, viruses and/or virus like particles, and the like substances and/or entities obtained from the cells. These may be considered as bioactive substances. The present method is mainly explained in the context of extracellular vesicles, more particularly exosomes, but the embodiments and examples may be applied to other cell-derived products as well.

The cell-derived products may comprise or be extracellular vesicles (EV). There are two main routes to get EVs: biofluids and cell culture. Using cell culture conditioned medium environment for EV production, for example exosome, production, enables providing more controlled environment.

The main technical limitation of prior art technologies is the need to control environmental parameters within the reactors, such that the phenotype of the cell and obtained cell-derived products, such as extracellular vesicles, for example exosomes, does not change.

In the present methods and systems a three-dimensional cell system is provided enabling incubation and/or culturing cells to produce cell-derived products. The three-dimensional cell system stimulates the secretion of *in vivo* like cell-derived products, such as extracellular vesicles, so it is desired to use three-dimensional (3D) cell system. The amount of produced cell-derived products is therefore higher with 3D cell system compared to 2D systems. For example three-dimensional spheroid system increases exosome secretion from mesenchymal stem cells. It was observed that 3D system derived EVs showed significantly different profiles in terms of secretion dynamics and signalling molecular contents (RNAs and DNAs) compared to 2D system derived EVs. There have been however challenges to implement such three-dimensional cell culture in efficient and reliable way, especially in bioreactors and the like environments. Even with 3D systems the risk of phenotypic alterations at the cellular level due to shear stress has been an issue.

The cell viability and health may be monitored through the system and is enabled by the present steady in-flow of media and/or cells with nanostructured cellulose, with an equal and steady out-flow of cell-derived products of interest and waste products for analysis and downstream application.

Preservation, separation, harvest and recovery of the cell-derived products, cells and/or any other material such as cell culture material is desired. It was noticed that it is important to maintain and protect the structure of cells and cell-derived products, especially vesicle type of products or other fragile macromolecules, during the culturing and the further step including separating, harvesting and recovering. This was achieved by using the materials, systems and methods disclosed herein.

The present methods can be used to provide scalable large scale cell systems, such as stem cell systems, and methods for upstream production of EVs, such as exosomes. This enables delivering stable and potent products for therapeutic applications in large amounts. This is achieved by methods which maintain the mixture of nanostructured cellulose and cells and enable harvesting the cell-derived product rich medium. This may be done at a rate equal to addition of new medium. The solutions of the embodiments in practice stimulate the *in vivo* like exosomes secretion, the sustained upstream production of exosomes. The methods and cell systems disclosed and discussed herein aim to produce cell-derived product(s) of interest by incubating the cells, but also involve cell cultures and cell culturing. Therefore terms "cell culture" and "cell system" may be used interchangeably.

An additional limitation for scaling up cell cultures to produce exosomes is the continued heavy reliance on animal serum for optimal cell growth. For example, fetal bovine serum (FBS) is high in endogenous exosomes and, if not removed before cell culture, process-related impurities stemming from FBS contaminants may make their way into the final product, which, from a regulatory standpoint for a medical product, is completely unfavorable. Therefore, xeno-free culture medium components are desired, provided that they conserve comparable cell characteristics and exosome product attributes that might be expected to be of therapeutic grade.

Nanostructured cellulose is xeno-free product, and thus, highly suitable for clinical grade cell-derived product production. Cells can be efficiently immobilized in the nanostructured cellulose which acts as a carrier material, and cultured for long periods of time thus allowing efficient production and also release of cell-derived products of interest. For example mesenchymal stem cells (MSCs) are efficient mass producer of exosomes for drug delivery. The inventors have shown that nanostructured cellulose such as NFC is very suitable for MSC culturing.

Nanostructured cellulose hydrogel is also very suitable material for such 3D spheroid cultures. It was found out how to implement a bioreactor utilizing nanostructured cellulose and overcome issues discussed herein.

The present methods can be carried out by using the bioreactor presented herein, which may be a bioreactor system comprising the container and any further parts and substances such as ones disclosed herein. The bioreactor for extracting cell-derived products from cultured cells may comprise comprising
- a container,
- an inlet for inputting cell culture medium into the container,
- an outlet for outputting cell culture medium comprising cell-derived products from the container,
- the container comprising, or being connected to, a compartment comprising nanostructured cellulose configured to receive cells, or containing cells, said compartment comprising a first separating surface separating the nanostructured cellulose from the outlet and allowing cell culture medium comprising cell products to pass, and optionally comprising a further separating surface for exchange of gas.

The container may be any suitable container, and the size, volume and/or shape of the container may be selected to according to the size of the cultivation, type of cells, type of cell-derived products, type of nanostructured cellulose and/or the like properties. The container may be a vessel, a reactor, a bottle, a flask, a cell culture plate, a bag or the like, which enables 3D cell culturing. One or more containers may be used, for example two or more containers may be arranged in parallel. Cell culture in three dimensions (3D cell culturing) refers to cell culturing wherein cells are arranged in all three dimensions, which is intended to distinguish from conventional 2D cultures, wherein cells are arranged and cultured as a layer, for example on a Petri dish or the like surface. 3D cell culture is commonly implemented by using plastic scaffolds, but in the present method the cell culture is carried out in a matrix comprising or consisting of nanostructured cellulose. It was found out that nanostructured cellulose hydrogel or entities formed from nanostructured cellulose are especially suitable for culturing cells for cell-derived products, so that the cell-derived products could be efficiently produced and also released, separated and harvested from the cell culture.

The inlet for inputting cell culture medium into the container or an compartment may be different from the outlet for extracting cell culture medium comprising cell-derived products from the container. The inlet and/or the outlet may be formed as a physical inlet and/or outlet in the container, for example as a tube, a valve, an aperture or combination thereof formed, embedded or connected into the container or the compartment. The inlet and/or the outlet may be connected or connectable to a tube, a hose, or other suitable way of conveying liquid and/or solid medium to and/or from the container, the reactor and/or the compartment. A flow of liquid, such as cell culture medium, which may contain other substances, such as nanostructured cellulose and/or cell-derived products, may be arranged to the inlet and/or from the outlet, preferably by using means for conveying liquid and/or means for providing a flow of liquid, for example by using a pump or the like device. This enables continuous production of cell-derived products. When a flow of liquid, such as the medium, is provided, the cells can be cultured and/or maintained in such a state which enables stable production of biologics. Without the flow the cells could be at a static state, wherein the cells and the products produced by the cells would degrade and/or change, so the production would not be stable and the spectre of cell-derived products would change over time.

The container may comprise a compartment for the nanostructured cellulose, or a compartment comprising the nanostructured cellulose, or the container may be connected such a compartment which is separate, for example by using suitable connecting means such as tube(s), hose(s) or the like. This compartment may be called as the first compartment, and it may be connected to second and/or further compartment(s) or other part(s), such as inlet(s) and/or outlet(s). The first compartment may comprise a fixed bed of the nanostructured cellulose. A second compartment may be a compartment separated from the first compartment by the first separating surface and optionally by one or more further surfaces. The second compartment is arranged to receive the separated cell-derived products from the first compartment, and the outlet may be directly connected to the second compartment.

The nanostructured cellulose configured to receive cells refer to nanostructured cellulose which is in a form suitable for receiving cells, as disclosed herein. More particularly the nanostructured cellulose shall be able to receive cells in such way that it can bind, adhere, embed, encapsulate, protect, support and/or maintain the cells, as discussed herein. For example the nanostructured cellulose may be configured to receive cells to embed them, preferably in such way that the cells are not released. Such nanostructured cellulose may have suitable porosity, fibril dimension such as diameter and/or ratio, nanostructural type, concentration, modification degree such as chemical, enzymatic and/or mechanical modification degree and/or type, such as fibrillation degree, one or more rheological property and the like properties, or combinations thereof.

In one embodiment the compartment comprising nanostructured cellulose and cells is a separate unit to the container. In one embodiment the compartment comprising nanostructured cellulose and cells is inside the container. The compartment comprising nanostructured cellulose and cells may be or comprise a fixed bed reactor, or a fixed bed reactor may comprise the compartment comprising nanostructured cellulose and cells.

The compartment comprises a first surface 20, which may be called as an exposing surface or a separating surface, such as a first separating surface, and which is designed to provide cell culture medium comprising cell-derived products from the cells to the outlet and/or to another compartment. The first surface therefore is arranged to allow cell culture medium and cell-derived products to pass, but preferably prevents the cells and the nanostructured cellulose, especially in the form provided in the compartment, to pass. The first surface may be a barrier, or it may act as a barrier, such as a selective barrier, which can keep the nanostructured cellulose and/or the cells in the compartment. The barrier may be a filter or it may act as a filter. The properties of the first surfaces may be arranged to specifically enable only cell-derived products if interest to pass. The first surface may be rigid or flexible. The first surface is designed and/or comprises means for controlling, such as limiting or selecting, the flow of substances from the first compartment to the outlet. This may be implemented by providing a membrane, a filter, a mesh or the like structure or layer with selective properties, such as selective permeability. The membrane may be a (fine) porous membrane, especially a low attachment membrane, for example ultra-low attachment membrane, which may comprise materials, for example as a coating, which inhibits nonspecific attachment of cells, biomolecules or other materials used in the present methods. For example a permeabilized and/or semi-permeable porous membrane may be provided, which is not permeable to the cells and/or nanostructured cellulose, which are located in the first compartment. However the (desired) cell-derived products can pass the membrane, usually in a liquid such as the cell culture medium, so they can be separated from the cells and conveyed to a different compartment and/or to the outlet, and recovered. A suitable filter which similar properties may be applied as well. The first surface may alternatively comprise a surface of the nanostructured cellulose, which can provide said selective properties, such as selective permeability. Such a specific nanostructured cellulose surface may be also provided as an additional selective surface. This may be obtained for example by providing a nanostructured cellulose hydrogel with suitable concentration, porosity and/or viscosity, which may be obtained by providing, using and/or preparing nanostructured cellulose with selected properties such as concentration, fibrillation degree, porosity, chemical composition and/or other modification degree, which may be obtained mechanically, chemically and/or enzymatically.

In one embodiment the first surface comprises a structure, which may be a layer, such as a membrane, a filter or a mesh, permeable to cell culture medium comprising the cell-derived products, and impermeable to cells and nanostructured cellulose. Such selected permeability may be obtained by selecting a cut-off size, such as a pore size or a mesh size of a membrane, a filter or other similar structure so that the cells and/or the nanostructured cellulose cannot pass the structure. The first surface may be also formed by the surface of the nanostructured cellulose itself, or such surface may be a further surface 30, such as a second or a third separating surface. If the nanostructured cellulose is provided as separate entities, the size (smallest diameter) of the entities is usually much higher than the size (smallest diameter) of the cells, so the cell diameter may be a limiting factor for selecting the pore or mesh size of the porous structure or layer, or other suitable limiting factor. In such case the pore size may be lower than the smallest average diameter of the cells. However, the cells may be cultured inside the nanostructured cellulose so they are not released to the cell culture medium, so in such case very large pore or mesh size can be used, especially if the nanostructured cellulose entities are large, for example in a form of large spherical forms, such as granules or beads, or elongated forms. Large cut-off size, such as pore or mesh size, enables better flow of liquid and substances, which facilitates cell culturing and producing and separating cell-derived products. The pore size may be in the range of 0.1-3 µm, such as 0.4-3 µm.

In one example the first surface comprises a membrane, a filter or a mesh having an average pore size in the range of 0.1-3 µm, such as 0.4-3 µm. The pore size may be an average pore size or an average pore diameter, which may be determined with microscope by using a suitable imaging software. This allows most cell-derived products, such as extracellular vesicles and smaller substances and molecules, to pass the membrane so they can be separated from the cells and nanostructured cellulose and finally recovered. Depending on the form of the nanostructured cellulose the pore size may be larger. For example if relatively large nanostructured cellulose entities are used, which may have a smallest diameter up to millimeters, a membrane, a mesh or other structure with pore size up to for example 100 µm, or even up to 300 µm or 500 µm, can be used, even with spherical or globular entities. With elongated entities, such as fibers or filaments it may be possible to use even larger pore size. Using a larger mesh, pore or aperture size of the dividing structure between the first and the second compartment allows better flow of medium through the structure, which facilitates the migration of substances and separation and recovery of the cell-derived products. It is possible to obtain mixing and/or flow of medium with a variety of means, such as by using suitable mixer 24, pump 40, and/or other actuator or by altering the volume of an compartment, for example by using a container with adjustable volume, for example a container equipped with a movable part, such as a deformable part, such as a bellow or the like, or a piston-like part such as a plunger, wherein the volume of the container is changed by operating the movable part, for example with an actuator connected to the movable part.

It is possible to select a suitable pore size, or cut-off size, for the first surface. With a suitable size it is possible to separate different cell-derived products according to their sizes. It is possible to provide a second or further surface(s) permeable to cell culture medium comprising the cell-derived products, and impermeable to cells and nanostructured cellulose. Such surface(s) may have a different cut-off or pore size, so they have a different permeability to cell-derived products. By providing a first surface, a second surface and optionally a suitable number of further surface(s) each having a different pore or cut-off size it is possible to separate the cell-derived products into different fractions according to their size. This would facilitate separating desired cell-derived products. The first and further surface may comprise similar or different structures, such as one or more membrane(s), one or more filter(s), which may comprise one or more ultrafilter(s), one or more mesh(es), and combinations thereof. These surfaces may be arranged in order capable of separating the cell-derived products according to their sizes, such as by providing a surface with a highest permeability (with the highest cut-off size or pore size) first, and then providing further surfaces in order presenting lowering permeabilities.

For example the average sizes, i.e. the smallest diameter, of extracellular vesicles may be mainly in the range of 50-300 nm, so it is possible to select one or more pore or cut-off size(s) for a first and optionally further separating surfaces for example from 500 nm, 400 nm, 300 nm, 250 nm, 200 nm, 150 nm, 100 nm and 50 nm. The first and further separating surfaces may define a second and further compartments, wherefrom the different fractions may be harvested.

In one embodiment the first surface is in a form of hollow fiber(s). In such case the reactor may be a hollow fiber reactor. The cells and the NFC may be on the surface of the fibers, and the cell-derived products are able to pass the wall of the fibers and enter the liquid flowing in the fibers, and are therefore separated from the cells and nanostructured cellulose and can be recovered.

The compartment may further comprise a further surface 30, which may be called as a gas exchange surface, and which is designed to allow exchange of gas(es). The gas exchange surface may be connected to or arranged in contact with a gas source and/or gas inlet and/or outlet. The gas exchange surface may be identical or similar to the first surface, for example it may comprise a membrane or the like structure. The gas exchange surface may be however specifically adapted for gas exchange, so for example pores or the like permeable structure may be adapted for gases. Therefore the pore size may be smaller compared to the first surface. The bioreactor may therefore comprises at the gas exchange surface a surface or membrane permeable to gases, and impermeable to cells and nanostructured cellulose, also preferably impermeable to liquid, such as aqueous liquid or solution.

The bioreactor, or the first compartment, may comprise means for mixing the nanostructured cellulose and cells in the compartment. The means for mixing may comprise a mixer, a stirrer, or the mixing may be implemented by providing suitable flow of liquid into and/or through the first compartment and/or the reactor. A mixer or a stirrer may comprise one or more suitable protruding part(s) for mixing the mixture, such as a rudder or the like, and preferably the mixing is arranged in such way that the cells and the nanostructured cellulose, and/or the cell-derived products, are not damaged. The mixing may be controlled, for example by controlling the speed of the mixer, and/or flow in the reactor or compartment.

In one embodiment the bioreactor comprises means for providing flow of cell culture medium via the inlet and/or the outlet, preferably through the (first) compartment. The cells are retained in the nanostructured cellulose in the compartment, and the (continuous) flow is arranged through the cells, the nanostructured cellulose and/or the compartment. In this way it is possible to obtain a perfusion bioreactor, a continuous bioreactor or a flow bioreactor, which terms may be used interchangeably herein, which is suitable for the purposes discussed herein, especially for continuous and stable production of cell-derived products. The means for providing flow of cell culture medium may comprise a pump, a mixer, a stirrer, a propeller, or the like actuator, which may be controllable, for example operatively connected to a control unit. The flow rate or speed may be controlled and it may be maintained in the desired level or range. The means for providing flow of cell culture medium may also comprise means for adjusting the volume of the container or an compartment. The means for providing the flow and the means for mixing may be the same means or they may be different means.

The bioreactor may comprise one or more sensor(s) for monitoring one or more parameter(s) from the bioreactor, for example from the first compartment and/or from the second or further compartment. The parameters may be one or more of temperature, pH, flow rate, conductivity, oxygen content, carbon dioxide content. The one or more sensor(s) may be operatively connected to a control unit. Therefore the control unit may be arranged to monitor the parameter(s) from the system, such as the bioreactor. The control unit may be arranged to carry out one or more controlling actions, which may be based on the one or more monitored parameter(s) and/or predetermined, such as programmed, actions.

The bioreactor may comprise one or more actuator(s) and/or actuators connected to devices for controlling the bioreactor, such as pumps, mixer(s), heater(s), aerator(s), means for adding a liquids and/or reagents, valve(s), agitator(s) and the like. The actuators and devices may be operatively connected to the control unit. The control unit may be configured to operate the actuators and devices, preferably to control the conditions in the bioreactor, especially as a feedback to one or more measured values or conditions, and/or as a response to one or more predetermined actions. For example the control unit may be programmed to carry out the cell culturing and/or other actions by the bioreactor according to a predetermined program, which may include maintaining for example the temperature in the reactor at a specified range, carrying out the culturing or other action(s) for a specified time period, maintaining a flow speed of medium at a specified range, maintaining pH, oxygen level and/or carbon dioxide level at a specified range and the like actions.

### Cells

The cells to be incubated and cultured in the bioreactor and with the present method can be any applicable cells, which may produce or be a source of cell-derived products of interest.

In general the cells may be cultured or incubated in the nanostructured cellulose, and they can be maintained and proliferated on or in the nanostructured cellulose without animal or human based agents or medium originating outside the cells. The cells may be evenly dispersed on or in the nanostructured cellulose hydrogel or the nanostructured cellulose entities. The cells incubated in the nanostructured cellulose for the production purposes disclosed herein form a cell system.

Initially the cells may be pre-cultured in a separate culture in a first medium, and recovered and transferred into a new medium, which may be similar or different than the first medium. A cell suspension is obtained. This may be combined and/or mixed with the nanostructured cellulose of the first compartment to obtain or form a cell system or a cell composition, which is a mixture of cells and nanostructured cellulose in selected form. When cells are cultured in the cell system a cell culture is formed, preferably a 3D system or culture which refers to a system or culture in the nanostructured cellulose, wherein the cells are permitted to grow and/or interact in all three dimensions. The nanostructured cellulose hydrogel matrix mimics the natural extracellular matrix structure and provides efficient transport of nutrients, gases and the like. When starting the cell incubation or culturing one or more propagation step(s) may be necessary to obtain a cell culture or a cell system which is optimal for production purposes. This may include inoculation and propagation of cells until a suitable cell density is obtained. After this the actual production can be initiated.

In one embodiment the cells are primary cells, such as stem cells. The cells may be eukaryotic cells, such as mammalian cells, for example human cells and/or non-human animal cells. In one embodiment the cells are human primary cells. In one embodiment the stem cells are human stem cells.

The term "cell culture" or "culturing of cells" may refer to one or more of maintaining, transporting, isolating, culturing, propagating, moving and/or differentiating of cells or tissues. The "cell system" may comprise the cell culture, but the cell system primarily aims to maintain the cells and to produce cell-derived products. It may be not desired to allow the cells to differentiate, and the propagation of the cells may be controlled and/or limited. It may also not be desired to transport, isolate and/or move the cells during the production. Cells may be maintained in any suitable arrangement, for example as individual cells, monolayers, cell clusters and/or spheroids, or as a tissue. In one embodiment the cells are present as aggregates, such as spheroids. The present nanostructured cellulose materials and arrangement thereof in the bioreactor facilitate maintaining the cell system, such as maintaining the cells in a desired arrangement and exhibiting a desired phenotype.

The cells, especially eukaryotic cells, may be stem cells or differentiated cells, such as cells originated or derived from human or animal body. Specific examples of cells include stem cells, undifferentiated cells, precursor cells, as well as fully differentiated cells and combinations thereof. In some examples the cells comprise cell types selected from the group consisting of keratocytes, keratinocytes, fibroblast cells, epithelial cells and combinations thereof. In some examples the cells are selected from the group consisting of stem cells, progenitor cells, precursor cells, connective tissue cells, epithelial cells, muscle cells, neuronal cells, endothelial cells, fibroblasts, keratinocytes, smooth muscle cells, stromal cells, mesenchymal cells, immune system cells, hematopoietic cells, dendritic cells, hair follicle cells and combinations thereof. The cells may be cancer cells or cancer stem cells. The cells may be genetically modified cells, such as transgenic cells, cisgenic cells or knock-out cells, or pathogenic cells. Such cells may be used for example for drug research or in therapeutical applications, such as for providing therapeutic substances. Especially stem cells may be used in therapeutical applications, and in such case it is especially important to protect the cells and the obtained cell-derived products during the process by using the materials, devices and methods disclosed herein.

Eukaryotic cells may be animal cells, such as mammalian cells. Examples of animal and mammalian cells include human cells, and non-human animal cells, such as mouse cells, rat cells, rabbit cells, monkey cells, pig cells, bovine cells, chicken cells and the like.

In one embodiment the cells are stem cells, such as omnipotent stem cells, which may be non-human, pluripotent, multipotent, oligopotent or unipotent stem cells. Stem cells are cells capable of renewing themselves through cell division and can differentiate into multi-lineage cells. These cells may be categorized as embryonic stem cells (ESCs), induced pluripotent stem cells (iPSCs), and adult stem cells, also called as tissue-specific or somatic stem cells. The stem cells may be human stem cells, which may be of non-embryonic origin, such as adult stem cells. These are undifferentiated cells found throughout the body after differentiation. They are responsible for e.g. organ regeneration and capable of dividing in pluripotent or multipotent state and differentiating into differentiated cell lineages. The stem cells may be human embryonic stem cell lines generated without embryo destruction, such as described for example in Cell Stem Cell. 2008 Feb 7;2(2):113-7. The stem cells may be obtained from a source of autologous adult stem cells, such as bone marrow, adipose tissue, or blood.

Examples of stem cells include mesenchymal stem cells (MSC), multipotent adult progenitor cells (MAPC^{®}), induced pluripotent stem cells (iPS), and hematopoietic stem cells.

In case of human stem cells the cells may be non-embryonic cells or embryonic cells, such as hESCs (human embryonic stem cells), which have been derived without destroying the embryo. In case of human embryonic stem cells the cells may be from a deposited cell line or made from unfertilized eggs, i.e. "parthenote" eggs or from parthenogenetically activated ovum, so that no human embryos are destroyed.

In one embodiment the cells are mesenchymal stem cells (MSC). Mesenchymal stem cells (MSCs) are adult stem cells which can be isolated from human and animal sources, such as from mammals. Mesenchymal stem cells are multipotent stromal cells that can differentiate into a variety of cell types, including osteoblasts, chondrocytes, myocytes and adipocytes. Mesenchyme itself is embryonic connective tissue that is derived from the mesoderm and that differentiates into hematopoietic and connective tissue. However mesenchymal stem cells do not differentiate into hematopoietic cells.

The terms mesenchymal stem cell and marrow stromal cell have been used interchangeably for many years, but neither term is sufficiently descriptive. Stromal cells are connective tissue cells that form the supportive structure in which the functional cells of the tissue reside. While this is an accurate description for one function of MSCs, the term fails to convey the relatively recently discovered roles of MSCs in the repair of tissue. The term encompasses multipotent cells derived from other non-marrow tissues, such as placenta, umbilical cord blood, adipose tissue, adult muscle, corneal stroma or the dental pulp of deciduous baby teeth. The cells do not have the capacity to reconstitute an entire organ

The International Society for Cellular Therapy has proposed minimum criteria to define MSCs. These cells (a) should exhibit plastic adherence, (b) possess specific set of cell surface markers, i.e. cluster of differentiation (CD)73, D90, CD105 and lack expression of CD14, CD34, CD45 and human leucocyte antigen-DR (HLA-DR) and (c) have the ability to differentiate in vitro into adipocyte, chondrocyte and osteoblast. These characteristics are valid for all MSCs, although few differences exist in MSCs isolated from various tissue origins. MSCs are present not only in fetal tissues but also in many adult tissues with few exceptions. Efficient population of MSCs has been reported from bone marrow. Cells which exhibit characteristics of MSCs have been isolated from adipose tissue, amniotic fluid, amniotic membrane, dental tissues, endometrium, limb bud, menstrual blood, peripheral blood, placenta and fetal membrane, salivary gland, skin and foreskin, sub-amniotic umbilical cord lining membrane, synovial fluid and Wharton's jelly.

Human mesenchymal stem cells (hMSC) display a very high degree of plasticity and are found in virtually all organs with the highest density in bone marrow. hMSCs serve as renewable source for mesenchymal cells and have pluripotent ability of differentiating into several cell lineages, including osteoblasts, chondrocytes, adipocytes, skeletal and cardiac myocytes, endothelial cells, and neurons in vitro upon appropriate stimulation, and in vivo after transplantation.

In one example the cells are multipotent adult progenitor cells (MAPC), which are derived from a primitive cell population that can be harvested from bone marrow, muscle and brain. MAPC are a more primitive cell population than mesenchymal stem cells, whilst they imitate embryonic stem cells characteristics they still retain adult stem cells potential in cell therapy. In vitro, MAPC demonstrated a vast differentiation potential to adipogenic, osteogenic, neurogenic, hepatogenic, hematopoietic, myogenic, chondrogenic, epithelial, and endothelial lineages. A key feature of MAPC is that they show large proliferative potential *in vitro* without losing their phenotype. MAPC may be used for treating a variety of diseases such as ischaemic stroke, graft versus host disease, acute myocardial infarct, organ transplant, bone repair and myelodysplasia. MAPC also enhance bone formation, promote neovascularisation, and have immunomodulatory effects.

Induced pluripotent stem cells (iPS) are a type of pluripotent stem cell that can be generated directly from adult cells. They can propagate practically indefinitely and may give rise to every other cell type in the body, including neurons, heart, pancreatic and liver cells. Induced pluripotent stem cells can be derived directly from adult tissues and they can be made in a patient-matched manner specific cell-derived products may be obtained which can be used without the risk of immune rejection. Human induced pluripotent stem cells are of special interest, and they can be generated from for example human fibroblasts, keratinocytes, peripheral blood cells, renal epithelial cells or other suitable cell types.

Hematopoietic stem cells (HSCs), also called as blood stem cells, are cells that can develop into all types of blood cells, including white blood cells, red blood cells, and platelets. Hematopoietic stem cells are found in the peripheral blood and the bone marrow. HSCs give rise to both the myeloid and lymphoid lineages of blood cells. Myeloid and lymphoid lineages both are involved in dendritic cell formation. Myeloid cells include monocytes, macrophages, neutrophils, basophils, eosinophils, erythrocytes, and megakaryocytes to platelets. Lymphoid cells include T cells, B cells, and natural killer cells.

The stem cells may be singe or separate stem cells, which are not aggregated, or they may be aggregated, such as stem cell spheroids, such as stem cell derived spheroids.

Cell spheroids refer to multicellular cell aggregates linked together by extracellular matrix, in this case multicellular cell aggregates linked together by nanostructured cellulose matrix. Spheroids are more complex than single cells present as separate cells due to dynamic cell-cell and cell-matrix interaction which makes them an important tool for resembling the *in vivo* tissues microenvironment *in vitro.* Cell spheroids can be formed by culturing or incubating cells in a matrix material to form three-dimensional cell culture system(s) containing multicellular aggregates or spheroids. When nanofibrillar cellulose was used as the matrix material cell spheroids could be obtained already after three days of culturing or incubating. In general the diameter of cell spheroids may vary and range from tens of micrometres to over millimetre. However herein cell spheroids with a controlled diameter suitable for cell-derived product producing purposes could be obtained by controlling and adjusting the culturing and matrix formation materials and conditions. The cells may be pre-cultured in a separate culture in a first medium, and they are then formed into aggregates in the nanostructured cellulose, such as nanofibrillar cellulose, such as in the first compartment, and/or the cell aggregates are maintained in the nanostructured cellulose in the first compartment.

In the cell-derived product producing process the multicell aggregates stabilized by the fibrillar cellulose network facilitated the activity, proliferation and differentiation of the cells. The nanostructured cellulose matrix created optimal conditions for such cell aggregates. The cell aggregates formed in the methods disclosed herein may be in the form of cell spheroids, or the cell aggregates may form cell spheroids.

The present application discloses a method for preparing cell composition, which may be used for producing cell-derived products, the method comprising
- culturing eukaryotic cells at conditions allowing the cells to form cell aggregates,
- providing the cell aggregates in a nanostructured cellulose hydrogel and/or combining the cell aggregates with a nanostructured cellulose hydrogel to obtain a cell system, such as a cell composition or a mixture, comprising eukaryotic cells in a nanostructured cellulose hydrogel. The cell system may be formed and/or may be located in the first compartment.

The method may comprise culturing the cells at conditions allowing the cells to coalesce. The conditions include suitable time to allow the coalescing and/or the formation of the aggregates. The conditions may include a suitable culturing medium, such as liquid medium and/or gel medium. The culturing may be carried out in a culture dish or plate, or in a multiwell microplate. The cells may be cultured in nanostructured cellulose hydrogel, such as nanofibrillar cellulose hydrogel, which preferably contains liquid culturing medium. Using nanostructured cellulose hydrogel may facilitate obtaining cell spheroids of desired size. The concentration of the hydrogel shall not be too high as too high concentration of the nanostructured cellulose gel matrix material, such as NFC, may prevent spheroid formation, especially with stem cells, such as pluripotent stem cells. Suitable concentration of the hydrogel may depend on the type of nanostructured cellulose used, and in may be in the range of 0.1-8% (w/w), such as 0.2-6% (w/w). Preferably the concentration of the hydrogel is not more than 3% (w/w), or not more than 2.5% (w/w) for nanofibrillar cellulose. In examples the cells are cultured in about 1% (w/w) NFC hydrogel, in about 1.5% (w/w) NFC hydrogel or in about 2% (w/w) NFC hydrogel. Certain cell types prefer low hydrogel concentrations as optimal culture conditions, such as 0.1-0.8% (w/w). For example MSCs may be cultured as embedded in 0.2-0.5% (w/w) hydrogel. If nanocrystalline cellulose is used the concentration may be higher, up to 5%, up to 6%, up to 7% or even up to 8%.

The method may comprise culturing the eukaryotic cells in nanofibrillar cellulose hydrogel having a concentration in the range of 0.1-3% (w/w), 0.2-2.2% (w/w), 0.1-2%, 0.2-2% (w/w), such as 0.1-0.8% (w/w), or 0.8-3% (w/w), 1.3-2.2% (w/w), 0.8-2%, 1-2% (w/w), such as 0.8-1.5% (w/w), or about 1% (w/w), about 1.5% (w/w) or about 2% (w/w). The cells may be seeded or provided to the cell culture in a density of 1x10⁵ cells/ml - 1x10⁷ cells/ml, such as in a density of 1x10⁶ - 5x10⁶ cells/ml. The same concentrations may be also used for single or separate cells, which may or may not be stem cells, and also when nanocrystalline cellulose is used.

The cell aggregates, which may be cell spheroids, may have an average diameter in the range of 80-700 µm, such as in the range of 80-300 µm. The diameter may be number-average diameter. It seems that cell spheroids generated with NFC, such as cultured in the NFC and/or combined with the NFC, having a concentration of about 2% (w/w) had a smaller average diameter, such as in the range of 80-250 µm, than cell spheroids generated in NFC with a lower concentration. The diameter could also be maintained. This may increase the survival of the cells *ex-vivo* and *in-vivo* and enables providing efficient and viable cell preparations and cell-derived products. It is also possible to adjust the diameter of the formed or forming cell spheroids, for example to reduce the diameter of the cells by adding EDTA solution or the like complexing reagent.

The method may also comprise providing nanostructured cellulose hydrogel, such as nanofibrillar cellulose hydrogel, which may be for culturing and/or for producing cell-derived products. The cell aggregates may be provided in the hydrogel and/or combined with the hydrogel. The hydrogel may be mixed, preferably by mixing gently with a pipette tip or the like tool, to obtain an even distribution of cells and hydrogel, and to avoid breaking the cells or cell spheroids and to avoid bubble formation.

The formed cell aggregates, which are multicellular aggregates, are herein called cell spheroids. The culturing may be carried out for at least three days, such as for 3-14 days, or for 3-7 days. Especially the method is suitable for stem cells. After the formation of aggregates, the cells may be harvested and washed with a suitable medium or buffer and/or suspended in to a suitable medium or buffer, such as a buffer or medium having a pH in the range of 6-8 as disclosed in previous. After this the cell aggregates may be transferred to the NFC hydrogel. The NFC hydrogel will form an interstitial matrix between the cell aggregates, which matrix reinforces the structure, immobilizes the cells but enables flow of agents though the matrix, which enables for example cell signalling, flow of nutrients and other important functions. The matrix of the embodiments refers to a matrix surrounding cells and forming a porous three-dimensional lattice, which is functionally similar to the interstitial matrix found in extracellular matrices is tissues. The matrix may be evenly distributed in the composition and/or between the cells. The matrix may be formed and/or further developed, i.e. the NFC may react with the cells to form the matrix, after the cells have been present in the matrix for a suitable period of time, such as at least for 6 hours, at least for 12 hours, or at least for 24 hours. The cells may be stored and/or incubated in the nanostructured cellulose hydrogel for several days or even for weeks.

It was found out that nanofibrillar cellulose, either chemically non-modified or chemically modified, especially anionically modified, promoted the formation of cell spheroids and stabilized them. The cell spheroids could be used for cell-derived product production. The NFC hydrogel used especially with stem-cell spheroids reduced further agglomeration of the cells and insulated cells from shearing forces during culturing and production in the bioreactor. The same effect is also present if the cells are present as separate, i.e. non-aggregated.

The cells and cell spheroids can be studied in a nanostructured cellulose hydrogel visually, for example microscopically, because of the optical properties of the hydrogel. Other tests can be also carried out while the cells are in the hydrogel matrix, as the matrix allows flow of molecular substances. The cells or cell spheroids can be released from the hydrogel by degrading the hydrogel enzymatically, for example by using one or more cellulase enzymes.

### Cell-derived products

The cell-derived products may be any applicable cell-derived products, such as one or more of the ones discussed herein. The cell-derived products may be any products derived from the cell, such as excreted and/or secreted by the cells, and/or otherwise released from the cells. The cells are not cell-derived products. The cell-derived products are preferably bioactive substances, which may be called in general as "biologics", so cell-derived products and biologics as used herein are interchangeable terms. It is desired to maintain the bioactivity of such substances.

The cell-derived products may be or comprise vesicles, such as extracellular vesicles. In one embodiment the cell-derived products are or comprise exosomes, preferably wherein the cells are mammalian cells. The cell-derived products may also be or comprise (macro)molecules, such as proteins, carbohydrates, lipids, nucleic acids, antibodies, hormones, other applicable molecules, such as recombinant molecules, and/or viruses or parts thereof. The cell-derived products may be or comprise a secretome, which according to one definition is a set of proteins expressed by an organism and secreted into the extracellular space.

In one example the cell-derived products comprise or are viruses, parts thereof, virus-like particles, viral products and/or viral vectors. These may be used for example for manufacture of vaccines or in gene therapy. In such case the cells are hosts cells for the viruses.

The cell-derived products may be or comprise bioactive substances, such as molecules and/or extracellular vesicles.

In one example the cell-derived products comprise or are proteins, such as therapeutic proteins, for example inti-inflammatory cytokines and other molecules, insuline, hormones etc.

In one example the cell-derived products are or comprise antibodies. In such case the method may be a method for producing, separating and/or recovering antibodies. It may be desired to use nanostructured cellulose entities for producing antibodies, especially by culturing the cells of top of the entities, so there is more surface available for the cells to grow. This may apply also for producing other cell-derived products disclosed herein.

The size of the cell-derived products may have an impact to the migration speed and efficiency in the nanostructured cellulose or other materials. For example vesicles, viruses and the like entities are larger than for example certain macromolecules, and as lipid monolayer or bilayer containing entities they may be fragile or otherwise such dynamic structures that the matrix material may have a substantial impact to the migration and maintenance of such cell-derived products. Therefore it may be desired to culture cell for such cell-derived products on the surface of the nanostructured cellulose entities and/or embedded in the entities, and/or use nanostructured cellulose of such type that allows the migration of the cell-derived products.

### Nanofibrillar cellulose

The nanostructured cellulose may comprise or be nanofibrillar cellulose. Nanofibrillar cellulose may be preferred for certain uses, for example when high aspect ratio and/or preserved fibrils are desired. These have an impact for example on the structure and the rheological properties of the material, and the ability to interact with the cells.

Preferably the concentration of the NFC hydrogel is not more than 3% (w/w), or not more than 2.5% (w/w). In examples the cells are incubated in about 1% (w/w) NFC hydrogel, in about 1.5% (w/w) NFC hydrogel or in about 2% (w/w) NFC hydrogel. The method may comprise incubating the cells in nanofibrillar cellulose hydrogel having a concentration in the range of 0.5-3% (w/w), 1-3% (w/w), 0.5-2.5% (w/w), 1-2.2% (w/w), 0.6-2%, 1-2% (w/w), such as 0.6-1.5% (w/w), or about 1% (w/w), about 1.5% (w/w) or about 2% (w/w). The cells may be seeded or provided to the cell system in a density of 1x10⁵ cells/ml - 1x10⁷ cells/ml, such as in a density of 1x10⁶ - 5x10⁶ cells/ml. It was found out that the concentration should not be lower as the viscosity of the hydrogel may be too low in concentration below 0.5% (w/w), or below 0.8% (w/w). On the other hand, the concentration shall not be too high, such as over 5% (w/w), or over 3% (w/w) or over 2.5% (w/w), because the flow of substances in and/or from the hydrogel matrix may decrease or even may be blocked. Already at a concentration over 2% (w/w), or over 2.5% (w/w), the pore size of the hydrogel tends to be too low, such as below 100 nm, and the hydrogel could be too stiff, which may cause trapping of the cell-derived products, especially larger cells products, such as vesicles and the like. Therefore too high concentration and too stiff or dense hydrogel may interfere the migration of the cell-derived products and/or the flow of the cell culture medium.

Said concentrations apply to NFC in hydrogel form, which may be present as substantially homogenous and/or continuous hydrogel, or to NFC formed into separate entities, as discussed herein. In the separate entities however higher concentrations may be used, such as up to 3% (w/w), 5% (w/w) or even up to 8% (w/w). Such higher concentrations may facilitate incubating the cells on the surface of the entities, or immediately below the surface, which may in turn facilitate the migration of substances to and from the entities to the surrounding medium. For example migration of large cell-derived products may be facilitated so that the production and recovery of the cell-derived products can be enhanced.

The nanofibrillar cellulose should have adequate degree of fibrillation so that the desired properties and effects are obtained. In one embodiment the nanofibrillar cellulose has a number average diameter of the fibrils and/or fibril bundles in the range of 1-200 nm. The nanofibrillar cellulose may be further characterized with rheological properties, such as viscosity and/or yield stress.

In one embodiment the nanofibrillar cellulose cellulose when dispersed in water, provides a zero shear viscosity in the range of 100-50000 Pa s, such as in the range of 300-8000 Pa s, and a yield stress in the range of 1-50 Pa, such as in the range of 2-15 Pa, determined by rotational rheometer at a consistency of 0.5% by weight in aqueous medium at 22±1 °C. Such material is fibrillated into such degree and has such properties that especially facilitated cell culturing, incubating, and the cell-derived product production. Particularly the zero shear viscosity in the range of 300-8000 Pa s and a yield stress in the range of 2-15 Pa are especially suitable for the present bioreactor applications. For example the cell-derived products were able to diffuse from the cell into the hydrogel and from the hydrogel into the medium so that they could be efficiently separated and recovered. In an especially advantageous embodiment the nanofibrillar cellulose comprises cellulose fibrils and/or fibril bundles having a number-average diameter in the range of 2-100 nm, such as 2-50 nm, 2-20 nm or 10-50 nm, and wherein the nanofibrillar cellulose, when dispersed in water, provides a yield stress in the range of 1-50 Pa, such as in the range of 2-15 Pa, determined by rotational rheometer at a consistency of 0.5% by weight in aqueous medium at 22±1 °C.

The nanofibrillar cellulose may be the only matrix material in the bioreactor and/or in the first compartment, and/or in the hydrogel and/or in the entities, such as the only polymeric material and/or the only cellulosic material. However, it is also possible to include other polymeric materials in addition to NFC, such as nanocrystalline cellulose, hyaluronan, hyaluronic acid and its derivates, peptide-based materials, proteins, other polysaccharides e.g. alginate or polyethylene glycol. Compositions forming a semi-interpenetrating network (semi-IPN) may be obtained, where nanofibrillar cellulose provides structural stability. The content of the other polymeric materials in the total composition as dry weight may be in the range of 20-80% (w/w), such as 40-60% (w/w), or 10-30% (w/w) or 10-20% (w/w).

The starting material for forming the hydrogel may be nanofibrillar cellulose, also called as nanocellulose, which refers to isolated cellulose fibrils and/or fibril bundles derived from cellulose raw material. Nanofibrillar cellulose is based on a natural polymer that is abundant in nature. Nanofibrillar cellulose has a capability of forming viscous hydrogel in water. Nanofibrillar cellulose production techniques may be based on disintegrating fibrous raw material, such as grinding of aqueous dispersion of pulp fibers to obtain nanofibrillated cellulose. After the grinding or homogenization process, the obtained nanofibrillar cellulose material is a dilute viscoelastic hydrogel.

The obtained material usually exists at a relatively low concentration homogeneously distributed in water due to the disintegration conditions. The starting material may be an aqueous gel at a concentration of 0.2-10% (w/w), for example 0.2-5% (w/w). The nanofibrillar cellulose may be obtained directly from the disintegration of fibrous raw material, such as cellulose fibers. Examples of commercially available nanofibrillar cellulose hydrogels include GrowDex^{®} variants by UPM.

Because of its nanoscale structure nanofibrillar cellulose has unique properties which enable functionalities which cannot be provided by conventional non-nanofibrillar cellulose or for example synthetic fibers or fibrils. It is possible to prepare materials and products which exhibit different properties than conventional products or products using conventional cellulosic materials or other polymeric materials. However, because of the nanoscale structure nanofibrillar cellulose is also a challenging material. For example dewatering or handling of nanofibrillar cellulose may be difficult.

The nanofibrillar cellulose may be prepared from cellulose raw material of plant origin, or it may also be derived from certain bacterial fermentation processes. The nanofibrillar cellulose is preferably made of plant material. Nanofibrillar cellulose is preferably obtained from plants by mechanical disintegration of cellulose fibers. The raw material may be based on any plant material that contains cellulose. In one example the fibrils are obtained from non-parenchymal plant material. In such case the fibrils may be obtained from secondary cell walls. One abundant source of such cellulose fibrils is wood fibres. The nanofibrillar cellulose may be manufactured by homogenizing wood-derived fibrous raw material, which may be chemical pulp. Cellulose fibers are disintegrated to produce fibrils which have an average diameter of only some nanometers, which is 200 nm or less in most cases, and gives a dispersion of fibrils in water. The fibrils originating from secondary cell walls are essentially crystalline with degree of crystallinity of at least 55%. Such fibrils may have different properties than fibrils originated from primary cell walls. For example the dewatering of fibrils originating from secondary cell walls may be more challenging. In general in the cellulose sources from primary cell walls, such as sugar beet, potato tuber and banana rachis, the microfibrils are easier to liberate from the fibre matrix than fibrils from wood, and the disintegration requires less energy. However, these materials are still somewhat heterogeneous and mainly consist of large fibril bundles.

Non-wood material may be from agricultural residues, grasses or other plant substances such as straw, leaves, bark, seeds, hulls, flowers, vegetables or fruits from cotton, corn, wheat, oat, rye, barley, rice, flax, hemp, manila hemp, sisal hemp, jute, ramie, kenaf, bagasse, bamboo or reed. The cellulose raw material could be also derived from the cellulose-producing micro-organism. The micro-organisms can be of the genus Acetobacter, Agrobacterium, Rhizobium, Pseudomonas or Alcaligenes, preferably of the genus Acetobacter and more preferably of the species Acetobacter xylinumor or Acetobacter pasteurianus.

It was found out that nanofibrillar cellulose obtained from plant cellulose, especially wood cellulose, is preferable for life science, medical or scientific products described herein. Wood cellulose is available in large amounts, and the preparation methods developed for wood cellulose enable further producing nanofibrillar materials suitable for such products. The nanofibrillar cellulose obtained by fibrillating plant fibers, especially wood fibers, differs structurally from nanofibrillar cellulose obtained from microbes, and it has different properties. For example compared to bacterial cellulose, nanofibrillated wood cellulose is homogenous and more porous and loose material, which is advantageous in applications involving living cells. Bacterial cellulose is usually used as such without similar fibrillation as in plant cellulose, so the material is different also in this respect. Bacterial cellulose is dense material which easily forms small spheroids and therefore the structure of the material is discontinuous, and it is not desired to use such material in the applications relating to living cells, especially when homogeneity of the material is required.

Nanofibrillar cellulose derived from plant material requires disintegrating cellulose fibers into fibrils and/or fibril bundles, which enables controlling the fibrillation process and the properties of the obtained fibrillated cellulose material. It is possible to modify the cellulose before the disintegration process, and control the fibrillation type and efficiency for example by selecting a suitable device, suitable process conditions and time, and other related parameters, to obtain nanofibrillar cellulose with desired properties such as fibrillation degree, aspect ratio and modification type. This is not possible with bacterial nanocellulose, which is usually present as already fibrillar form. Plant cellulose is also preferred for the present methods, wherein cell-derived products are prepared and it is desired to have a controlled production without any disturbing or contaminating substances. When using plant cellulose there would be no risk of presence of any substances of bacterial origin.

Wood may be from softwood tree such as spruce, pine, fir, larch, douglas-fir or hemlock, or from hardwood tree such as birch, aspen, poplar, alder, eucalyptus, oak, beech or acacia, or from a mixture of softwoods and hardwoods. In one example the nanofibrillar cellulose is obtained from wood pulp. The nanofibrillar cellulose may be obtained from hardwood pulp. In one example the hardwood is birch. The nanofibrillar cellulose may be obtained from softwood pulp. In one example said wood pulp is chemical pulp. Chemical pulp may be desired for the products disclosed herein. Chemical pulp is pure material and may be used in a wide variety of applications. For example chemical pulp lack the pitch and resin acids present in mechanical pulp, and it is more sterile or easily sterilisable. Further, chemical pulp is more flexible and provides advantageous properties for example in medical and scientific materials. For example very homogenous nanofibrillar cellulose materials may be prepared without excess processing or need for specific equipment or laborious process steps.

Nanofibrillar cellulose, including the cellulose fibrils and/or fibril bundles, is characterized by a high aspect ratio (length/diameter). The average length of nanofibrillar cellulose (the median length of particles such as fibrils and/or fibril bundles) may exceed 1 µm, and in most cases it is 50 µm or less. If the elementary fibrils are not completely separated from each other, the entangled fibrils, such as fibril bundles, may have an average total length for example in the range of 1-100 µm, 1-50 µm, or 1-20 µm. This applies especially for native grades of fibrils which are not shortened or digested, for example chemically, enzymatically or mechanically. However, if the nanofibrillar material is highly fibrillated, the elementary fibrils may be completely or almost completely separated and the average fibril length is shorter, such as in the range of 1-10 µm or 1-5 µm. Strongly derivatized nanofibrillar cellulose may have a shorter average fibril length, such as in the range of 0.3-50 µm, such as 0.3-20 µm, for example 0.5-10 µm or 1-10 µm. Especially shortened fibrils, such as enzymatically or chemically digested fibrils, or mechanically treated material, may have an average fibril length of less than 1 µm, such as 0.1-1 µm, 0.2-0.8 µm or 0.4-0.6 µm. The fibril length and/or diameter may be estimated microscopically, for example using CRYO-TEM, SEM or AFM images. A suitable imaging software may be used. If high aspect ratio is desired, it is not desired to shorten the fibril length so it may not be desired to use chemically or enzymatically digested cellulose, or such highly mechanically fibrillated material that the fibrils are already shortened. A low aspect ratio usually can be detected as decreased viscosity of NFC dispersion.

The average lengths and/or diameters disclosed herein may be number-average lengths and/or diameters, in regard of the NFC but also other particles, entities, cells, aggregates and the like discussed herein. These dimensions may be determined microscopically, as discussed herein. The term "fibrils" also includes fibril bundles, where applicable. The mechanically disintegrated cellulosic material may contain at least a small amount of cellulose which is not fully separated into elemental fibrils but is still in the form of fibril bundles.

The average diameter (width) of nanofibrillar cellulose is less than 1 µm, or 500 nm or less, such as in the range of 1-500 nm, but preferably 200 nm or less, even 100 nm or less or 50 nm or less, such as in the range of 1-200 nm, 2-200 nm, 2-100 nm, or 2-50 nm, even 2-20 for highly fibrillated material. The diameters disclosed herein refer to fibrils and/or fibril bundles. The smallest fibrils are in the scale of elementary fibrils, the average diameter being typically in the range of 4-12 nm. The dimensions and size distribution of the fibrils depend on the refining method and efficiency. In case of highly refined native nanofibrillar cellulose, the average fibril and/or fibril bundle diameter may be in the range of 2-200 nm or 2-100 nm, for example in the range of 10-50 nm. Nanofibrillar cellulose is characterized by a large specific surface area and a strong ability to form hydrogen bonds. In water dispersion, the nanofibrillar cellulose typically appears as either light or turbid gel-like material. Depending on the fiber raw material, nanofibrillar cellulose obtained from plants, especially wood, may also contain small amounts of other plant components, especially wood components, such as hemicellulose or lignin. The amount is dependent on the plant source.

In general cellulose nanomaterials may be divided into categories according to TAPPI W13021, which provides standard terms for cellulose nanomaterials. Not all of these materials are nanofibrillar cellulose. Two main categories are "Nano objects" and "Nano structured materials". Nanostructured materials include "Cellulose microcrystals" (sometimes called as CMC) having a diameter of 10-12 µm and length:diameter ratio (L/D) <2, and "Cellulose microfibrils" having a diameter of 10-100 nm and a length of 0.5-50 µm. Nano objects include "Cellulose nanofibers", which can be divided into "Cellulose nanocrystals" (CNC) having a diameter of 3-10 nm and L/D >5, and "Cellulose nanofibrils" (CNF or NFC), having a diameter of 5-30 nm and L/D >50.

Different grades of nanofibrillar cellulose may be categorized based on three main properties: (i) size distribution, length and/or diameter (ii) chemical composition, and (iii) rheological properties. To fully describe a grade, two or more properties may be used in parallel. Examples of different grades include native (chemically and/or enzymatically unmodified) NFC, oxidized NFC (high viscosity), oxidized NFC (low viscosity), carboxymethylated NFC and cationized NFC. Within these main grades, also sub-grades exist, for example: extremely well fibrillated vs. moderately fibrillated, high degree of substitution vs. low degree of substitution, low viscosity vs. high viscosity etc. The fibrillation technique and the chemical pre-modification have an influence on the fibril size distribution. Typically, non-ionic grades have wider average fibril and/or fibril bundle diameter (for example in the range of 10-100 nm, or 10-50 nm) while the chemically modified grades are a lot thinner (for example in the range of 2-20 nm). Distribution is also narrower for the modified grades. Certain modifications, especially TEMPO-oxidation, yield shorter fibrils.

In the chemical derivatization process a desired degree of substitution of the cellulose can be obtained. This may be carried out by controlling the chemical derivatization process, such as by selecting suitable raw material(s), reaction conditions, equipment, reaction time, reagents and/or the like properties. For example, TEMPO or N-oxyl mediated oxidation is typically conducted to charge values from 300 to 1500 micromol/g, preferably 600 to 1200 micromol/g, most preferably 700 to 1100 micromol/g. The oxidized NFC may contain also aldehyde functional groups, typically between 0 to 250 micromol/g. Derivatization via carboxymethylation is typically conducted for cellulose pulp to ds levels between 0.05 to 0.3, preferably between 0.08-0.25, most preferably 0.10-0.2 prior to fibrillation. If the derivatization is conducted by cationization, the DS levels are typically between 0.05 and 0.4, preferably 0.15-0.3.

Depending on the raw material source, e.g. hardwood vs. softwood pulp, different polysaccharide composition exists in the final nanofibrillar cellulose product. Commonly, the non-ionic grades are prepared from bleached birch pulp, which yields high xylene content (25% by weight). Modified grades are prepared either from hardwood or softwood pulps. In those modified grades, the hemicelluloses are also modified together with the cellulose domain. Most probably, the modification is not homogeneous, i.e. some parts may be more modified than others. Thus, detailed chemical analysis is usually not possible as the modified products are complicated mixtures of different polysaccharide structures.

In an aqueous environment, a dispersion of cellulose nanofibrils forms a viscoelastic hydrogel network. The gel is formed already at relatively low concentrations of for example 0.05-0.2% (w/w) by dispersed and hydrated entangled fibrils. The viscoelasticity of the NFC hydrogel may be characterized for example with dynamic oscillatory rheological measurements.

The nanofibrillar cellulose hydrogels exhibit characteristic rheological properties. For example they are shear-thinning or pseudoplastic materials, which may be considered as a special case of thixotropic behavior, which means that their viscosity depends on the speed or force by which the material is deformed. When measuring the viscosity in a rotational rheometer, the shear-thinning behavior is seen as a decrease in viscosity with increasing shear rate. The hydrogels show plastic behavior, which means that a certain shear stress (force) is required before the material starts to flow readily. This critical shear stress is often called the yield stress. The yield stress can be determined from a steady state flow curve measured with a stress controlled rheometer. When the viscosity is plotted as function of applied shear stress, a dramatic decrease in viscosity is seen after exceeding the critical shear stress. The zero shear viscosity and the yield stress are the most important rheological parameters to describe the suspending power of the materials. These two parameters separate the different grades quite clearly and thus enable classification of the grades.

The dimensions of the fibrils and/or fibril bundles, as well as other properties of the nanofibrillar cellulose, are dependent for example on the raw material, the disintegration method and number of disintegration runs. Mechanical disintegration of the cellulose raw material may be carried out with any suitable equipment such as a refiner, grinder, disperser, homogenizer, colloider, friction grinder, pin mill, rotor-rotor disperser, ultrasound sonicator, fluidizer such as microfluidizer, macrofluidizer or fluidizer-type homogenizer. The disintegration treatment is performed at conditions wherein water is sufficiently present to prevent the formation of bonds between the fibers.

In one example the disintegration is carried out by using a disperser having at least one rotor, blade or similar moving mechanical member, such as a rotor-rotor disperser, which has at least two rotors. In a disperser the fiber material in dispersion is repeatedly impacted by blades or ribs of rotors striking it from opposite directions when the blades rotate at the rotating speed and at the peripheral speed determined by the radius (distance to the rotation axis) in opposite directions. Because the fiber material is transferred outwards in the radial direction, it crashes onto the wide surfaces of the blades, i.e. ribs, coming one after the other at a high peripheral speed from opposite directions; in other words, it receives a plurality of successive impacts from opposite directions. Also, at the edges of the wide surfaces of the blades, i.e. ribs, which edges form a blade gap with the opposite edge of the next rotor blade, shear forces occur, which contribute to the disintegration of the fibers and detachment of fibrils. The impact frequency is determined by the rotation speed of the rotors, the number of the rotors, the number of blades in each rotor, and the flow rate of the dispersion through the device.

In a rotor-rotor disperser the fiber material is introduced through counter-rotating rotors, outwards in the radial direction with respect to the axis of rotation of the rotors in such a way that the material is repeatedly subjected to shear and impact forces by the effect of the different counter-rotating rotors, whereby it is simultaneously fibrillated. One example of a rotor-rotor disperser is an Atrex device.

Another example of a device suitable for disintegrating is a pin mill, such as a multi-peripheral pin mill. One example of such device includes a housing and in it a first rotor equipped with collision surfaces; a second rotor concentric with the first rotor and equipped with collision surfaces, the second rotor being arranged to rotate in a direction opposite to the first rotor; or a stator concentric with the first rotor and equipped with collision surfaces. The device includes a feed orifice in the housing and opening to the center of the rotors or the rotor and stator, and a discharge orifice on the housing wall and opening to the periphery of the outermost rotor or stator.

In one example the disintegrating is carried out by using a homogenizer. In a homogenizer the fiber material is subjected to homogenization by an effect of pressure. The homogenization of the fiber material dispersion to nanofibrillar cellulose is caused by forced through-flow of the dispersion, which disintegrates the material to fibrils. The fiber material dispersion is passed at a given pressure through a narrow through-flow gap where an increase in the linear velocity of the dispersion causes shearing and impact forces on the dispersion, resulting in the removal of fibrils from the fiber material. The fiber fragments are disintegrated into fibrils in the fibrillating step.

As used herein, the term "fibrillation" generally refers to disintegrating fiber material mechanically by work applied to the particles, where cellulose fibrils are detached from the fibers or fiber fragments. The work may be based on various effects, like grinding, crushing or shearing, or a combination of these, or another corresponding action that reduces the particle size. The expressions "disintegration" or "disintegration treatment" may be used interchangeably with "fibrillation".

The fiber material dispersion that is subjected to fibrillation is a mixture of fiber material and water, also herein called "pulp". The fiber material dispersion may refer generally to whole fibers, parts (fragments) separated from them, fibril bundles, or fibrils mixed with water, and typically the aqueous fiber material dispersion is a mixture of such elements, in which the ratios between the components are dependent on the degree of processing or on the treatment stage, for example number of runs or "passes" through the treatment of the same batch of fiber material.

One way to characterize the nanofibrillar cellulose is to use the viscosity of an aqueous solution or dispersion containing said nanofibrillar cellulose. The viscosity may be for example Brookfield viscosity or zero shear viscosity. The specific viscosity, as described herein, can be used to distinguish nanofibrillar cellulose from non-nanofibrillar cellulose.

The nanofibrillar cellulose may also be characterized by the average diameter (or width), or by the average diameter together with the viscosity, such as zero shear viscosity. In one example nanofibrillar cellulose suitable for use in the products described herein has a number average fibril diameter in the range of 1-200 nm, or 1-100 nm. In one example said nanofibrillar cellulose has a number average fibril diameter in the range of 1-50 nm, such as 2-20 nm or 5-30 nm. In one example said nanofibrillar cellulose has a number average fibril diameter in the range of 2-15 nm, such as in the case of TEMPO oxidized nanofibrillar cellulose.

The diameter of a fibril may be determined with several techniques, such as by microscopy. Fibril thickness and width distribution may be measured by image analysis of microscope images, such as images from a field emission scanning electron microscope (FE-SEM), a transmission electron microscope (TEM), such as a cryogenic transmission electron microscope (CRYO-TEM), or an atomic force microscope (AFM). In general AFM and TEM, especially CRYO-TEM, suit best for nanofibrillar cellulose grades with narrow fibril diameter distribution. From Cryo-TEM images, also the bundled structure can be seen.

Degree of fibrillation can be evaluated by using fiber analysis where number of larger, only partially fibrillated, entities are evaluated. For example, in the case of derivatized nanofibrillar cellulose the number of those particles per mg of dry sample may be in the range of 0-10000, such as in the range of 0-5000, for example in the range of 0-1000. However, in non-derivatized NFC the number of non-fibrillated particles/mg is typically somewhat higher in the range of 0-20000, such as in the range of 0-10000, for example in the range of 0-5000. The fiber analysis may be carried out using Fiberlab method.

The stiffness of the nanofibrillar cellulose hydrogels can be evaluated from viscoelastic measurements of the gels. Typically the storage modulus for 0.5% (by weight) nanofibrillar cellulose hydrogel in pure water at pH 7 at 25±1 °C or 22±1°C is between 1 to 50 Pa, preferably 2 to 20 Pa. Often the derivatized NFC builds up stiffer hydrogels, but extensive fibrillation of these grades may lead also to lower storage modulus.

A rheometer viscosity of the nanofibrillar cellulose dispersion may be measured according to one example at 22°C with a stress controlled rotational rheometer (AR-G2, TA Instruments, UK) equipped with a narrow gap vane geometry (diameter 28 mm, length 42 mm) in a cylindrical sample cup having a diameter of 30 mm. After loading the samples to the rheometer they are allowed to rest for 5 min before the measurement is started. The steady state viscosity is measured with a gradually increasing shear stress (proportional to applied torque) and the shear rate (proportional to angular velocity) is measured. The reported viscosity (=shear stress/shear rate) at a certain shear stress is recorded after reaching a constant shear rate or after a maximum time of 2 min. The measurement is stopped when a shear rate of 1000 s⁻¹ is exceeded. This method may be used for determining the zero-shear viscosity.

In another example rheological measurements of the hydrogel samples were carried out with a stress controlled rotational rheometer (AR-G2, TA instruments, UK) equipped with 20 mm plate geometry. After loading the samples to the rheometer, 1 mm gap, without dilution, they were allowed to settle for 5 min before the measurement was started. The stress sweep viscosity was measured with gradually increasing shear stress in a range of 0,001-100 Pa at the frequency 10 rad/s, strain 2%, at 25°C. Storage modulus, loss modulus and yield stress/fracture strength can be determined.

In one example the nanofibrillar cellulose, for example provided as a starting material in the method, when dispersed in water, provides a zero shear viscosity ("plateau" of constant viscosity at small shearing stresses) in the range of 1000-100000 Pa·s, such as in the range of 5000-50000 Pa·s, and a yield stress (shear stress where the shear thinning begins) in the range of 1-50 Pa, such as in the range of 2-15 Pa, determined by rotational rheometer at a consistency of 0.5% (w/w) by weight in aqueous medium at 22±1°C. Such nanofibrillar cellulose may also have an average fibril diameter of 200 nm or less, such as in the range of 1-200 nm.

Turbidity is the cloudiness or haziness of a fluid caused by individual particles (total suspended or dissolved solids) that are generally invisible to the naked eye. There are several practical ways of measuring turbidity, the most direct being some measure of attenuation (that is, reduction in strength) of light as it passes through a sample column of water. The alternatively used Jackson Candle method (units: Jackson Turbidity Unit or JTU) is essentially the inverse measure of the length of a column of water needed to completely obscure a candle flame viewed through it.

Turbidity may be measured quantitatively using optical turbidity measuring instruments. There are several commercial turbidometers available for measuring turbidity quantitatively. In the present case the method based on nephelometry is used. The units of turbidity from a calibrated nephelometer are called Nephelometric Turbidity Units (NTU). The measuring apparatus (turbidometer) is calibrated and controlled with standard calibration samples, followed by measuring of the turbidity of the diluted NFC sample.

In one turbidity measurement method, a nanofibrillar cellulose sample is diluted in water, to a concentration below the gel point of said nanofibrillar cellulose, and turbidity of the diluted sample is measured. Said concentration where the turbidity of the nanofibrillar cellulose samples is measured is 0.1%. HACH P2100 Turbidometer with a 50 ml measuring vessel is used for turbidity measurements. The dry matter of the nanofibrillar cellulose sample is determined and 0.5 g of the sample, calculated as dry matter, is loaded in the measuring vessel, which is filled with tap water to 500 g and vigorously mixed by shaking for about 30 s. Without delay the aqueous mixture is divided into 5 measuring vessels, which are inserted in the turbidometer. Three measurements on each vessel are carried out. The mean value and standard deviation are calculated from the obtained results, and the final result is given as NTU units.

One way to characterize nanofibrillar cellulose is to define both the viscosity and the turbidity. Low turbidity refers to small size of the fibrils, such as small diameter, as small fibrils scatter light poorly. In general as the fibrillation degree increases, the viscosity increases and at the same time the turbidity decreases. This happens, however, until a certain point. When the fibrillation is further continued, the fibrils finally begin to break and shorten, and therefore cannot form a strong network any more. Therefore, after this point, both the turbidity and the viscosity begin to decrease.

In one example the turbidity of anionic nanofibrillar cellulose is lower than 90 NTU, for example from 3 to 90 NTU, such as from 5 to 60, for example 8-40 measured at a consistency of 0.1% (w/w) in aqueous medium, and measured by nephelometry. In one example the turbidity of native nanofibrillar may be even over 200 NTU, for example from 10 to 220 NTU, such as from 20 to 200, for example 50-200 measured at measured at 20°C±1°C a consistency of 0.1% (w/w) in aqueous medium, and measured by nephelometry. To characterize the nanofibrillar cellulose these ranges may be combined with the viscosity ranges of the nanofibrillar cellulose, such as zero shear viscosity, storage modulus and/or yield stress.

Nanofibrillar cellulose may be or comprise non-modified nanofibrillar cellulose. The drainage of non-modified nanofibrillar cellulose is significantly faster than for example anionic grade. Non-modified nanofibrillar cellulose generally has a Brookfield viscosity in the range of 2000-10000 mPa·s, measured at 20°C±1°C, at a consistency of 0.8% (w/w) and at 10 rpm.

The disintegrated fibrous cellulosic raw material may be modified fibrous raw material. Modified fibrous raw material means raw material where the fibers are affected by the treatment so that cellulose nanofibrils are more easily detachable from the fibers. The modification is usually performed to fibrous cellulosic raw material which exists as a suspension in a liquid, i.e. pulp.

The modification treatment to the fibers may be chemical, enzymatic or physical. In chemical modification the chemical structure of cellulose molecule is changed by chemical reaction ("derivatization" of cellulose), preferably so that the length of the cellulose molecule is not affected but functional groups are added to β-D-glucopyranose units of the polymer. The chemical modification of cellulose takes place at a certain conversion degree, which is dependent on the dosage of reactants and the reaction conditions, and as a rule it is not complete so that the cellulose will stay in solid form as fibrils and does not dissolve in water. In physical modification anionic, cationic, or non-ionic substances or any combination of these are physically adsorbed on cellulose surface.

The cellulose in the fibers may be especially ionically charged after the modification. The ionic charge of the cellulose weakens the internal bonds of the fibers and will later facilitate the disintegration to nanofibrillar cellulose. The ionic charge may be achieved by chemical or physical modification of the cellulose. The fibers may have higher anionic or cationic charge after the modification compared with the starting raw material. Most commonly used chemical modification methods for making an anionic charge are oxidation, where hydroxyl groups are oxidized to aldehydes and carboxyl groups, sulphonization and carboxymethylation. Chemical modifications introducing groups, such as carboxyl groups, which may take part in forming a covalent bond between the nanofibrillar cellulose and the bioactive molecule, may be desired. A cationic charge in turn may be created chemically by cationization by attaching a cationic group to the cellulose, such as quaternary ammonium group.

Nanofibrillar cellulose may comprise chemically modified nanofibrillar cellulose, such as anionically modified nanofibrillar cellulose or cationically modified nanofibrillar cellulose. In one example the nanofibrillar cellulose is anionically modified nanofibrillar cellulose. In one example the anionically modified nanofibrillar cellulose is oxidized nanofibrillar cellulose. In one example the anionically modified nanofibrillar cellulose is sulphonized nanofibrillar cellulose. In one example the anionically modified nanofibrillar cellulose is carboxymethylated nanofibrillar cellulose. The material obtained with the anionical modification of cellulose may be called anionic cellulose, which refers to material wherein the amount or proportion of anionic groups, such as carboxylic groups, is increased by the modification, when compared to a non-modified material. It is also possible to introduce other anionic groups to the cellulose, instead or in addition to carboxylic groups, such as phosphate groups or sulphate groups. The content of these groups may be in the same ranges as is disclosed for carboxylic acid herein.

It may be desired that the nanofibrillar cellulose has a suitable carboxylic acid content, such as in the range of 0.6-1.4 mmol COOH/g, for example in the range of 0.7-1.2 mmol COOH/g, or in the range of 0.7-1.0 mmol COOH/g or 0.8-1.2 mmol COOH/g, determined by conductometric titration.

The cellulose may be oxidized. In the oxidation of cellulose, the primary hydroxyl groups of cellulose may be oxidized catalytically by a heterocyclic nitroxyl compound, such as through N-oxyl mediated catalytic oxidation, for example 2,2,6,6-tetramethylpiperidinyl-1-oxy free radical, generally called "TEMPO". The primary hydroxyl groups (C6-hydroxyl groups) of the cellulosic β-D-glucopyranose units are selectively oxidized to carboxylic groups. Some aldehyde groups are also formed from the primary hydroxyl groups. Regarding the finding that low degree of oxidation does not allow efficient enough fibrillation and higher degree of oxidation inflicts degradation of cellulose after mechanical disruptive treatment, the cellulose may be oxidized to a level having a carboxylic acid content in the oxidized cellulose in the range of 0.5-2.0 mmol COOH/g pulp, 0.6-1.4 mmol COOH/ g pulp, or 0.8-1.2 mmol COOH / g pulp, preferably to 1.0-1.2 mmol COOH/ g pulp, determined by conductometric titration. When the fibers of oxidized cellulose so obtained are disintegrated in water, they give stable transparent dispersion of individualized cellulose fibrils, which may be, for example, of 3-5 nm in width. With oxidized pulp as the starting medium, it is possible to obtain nanofibrillar cellulose where Brookfield viscosity measured at a consistency of 0.8% (w/w) is at least 10000 mPa·s, for example in the range of 10000-30000 mPa·s.

Whenever the catalyst "TEMPO" is mentioned in this disclosure, it is evident that all measures and operations where "TEMPO" is involved apply equally and analogously to any derivative of TEMPO or any heterocyclic nitroxyl radical capable of catalyzing selectively the oxidation of the hydroxyl groups of C6 carbon in cellulose.

Auxiliary agents for enhancing the manufacturing process or improving or adjusting the properties of the NFC or products formed from the NFC may be included in the nanofibrillar cellulose dispersion. Similar agents may be included also in nanocrystalline cellulose. Such auxiliary agents may be soluble in the liquid phase of the dispersion, they may form an emulsion or they may be solid. Auxiliary agents may be added already during the manufacturing of the nanofibrillar cellulose dispersion to the raw material or they may be added to a formed nanofibrillar cellulose dispersion or gel. The auxiliary agents may be also added to the NFC product, i.e. the entities, for example by impregnating, spraying, dipping, soaking or the like method. The auxiliary agents are usually not covalently bound to the nanofibrillar cellulose, so they may be releasable from the nanocellulose matrix. A controlled and/or sustained release of such agents may be obtained when using NFC as matrix. Examples of auxiliary agents include therapeutic (pharmaceutic) agents and other agents affecting to the properties of the nanofibrillar cellulose or to the properties of the cells and/or cell products, such as buffers, surfactants, plasticizers, emulsifiers, bioactive agents or the like. In one example the dispersion contains one or more salts, which may be added to enhance the properties of the final product. Examples of salts include chloride salts, such as sodium chloride, calcium chloride and potassium chloride. The salt may be included in an amount in the range of 0.01-1.0% (w/w) of the dry matter in the dispersion. The NFC product may also be dipped or soaked in a solution of sodium chloride, such as in an aqueous solution of about 0.9% sodium chloride. Desired salt content in the NFC product may be in the range of 0.5-1%, such as about 0.9%, of the volume of the wet product. The salts, buffers and the like agents may be provided to obtain physiological conditions.

Multivalent cations may be included to obtain non-covalent crosslinking of the nanofibrillar cellulose. One example provides a nanofibrillar cellulose product comprising nanofibrillar cellulose, especially comprising anionically modified nanofibrillar cellulose, and multivalent cations, such as multivalent metal cations, for example selected from cations of calcium, barium, magnesium, zinc, aluminum, gold, platinum and titanium, wherein the nanofibrillar cellulose is crosslinked by the multivalent cations. Especially barium and calcium may be useful in biomedical application, and especially barium may be used in labelling. The amount of the multivalent cations may be in the range of 0.1-3% (w/w), for example 0.1-2% (w/w) calculated from the dry content of the hydrogel.

One example provides a method for preparing such a hydrogel, the method comprising providing pulp, disintegrating the pulp until nanofibrillar cellulose is obtained, forming the nanofibrillar cellulose into a hydrogel

The nanofibrillar cellulose may be fibrillated into a desired fibrillation degree and adjusted into desired water content, or otherwise modified, so that it forms a hydrogel having desired properties as described herein. In one example the nanofibrillar cellulose in the hydrogel is anionically modified nanofibrillar cellulose.

The hydrogel to be used in the present applications is preferably homogenous. Therefore the method for preparing the hydrogel may include homogenizing a hydrogel comprising nanofibrillar cellulose, preferably with a homogenizing device such as ones described herein. With this preferably non-fibrillating homogenizing step it is possible to remove areas of discontinuity from the gel. A homogenous gel having better properties for the applications is obtained. The hydrogel may be further sterilized, for example by using heat and/or radiation, and/or by adding sterilizing agents, such as antimicrobials. The hydrogels obtained as discussed in previous may be used for forming the entities.

The present application discloses use of nanofibrillar cellulose for preparing the hydrogels or entities disclosed herein. The nanofibrillar cellulose may be any suitable nanofibrillar cellulose disclosed herein.

### Nanocrystalline cellulose

Nanocrystalline cellulose is different material from nanofibrillar cellulose, and has different properties and behaviour. Nanocrystalline cellulose is produced from cellulose by acid hydrolysis, which removes the amorphous region to obtain the crystalline region of cellulose. Therefore nanocrystalline cellulose practically consists of crystalline cellulose, and it lacks amorphous regions of cellulose. The fibril length is substantially shortened. Nanofibrillar cellulose on the other hand includes both crystalline parts as straight segments and amorphous parts, which provide a kink in the fibril. Nanofibrillar cellulose is not produced from cellulose by acid hydrolysis. Nanofibrillar cellulose as used herein is also not meant to include cellulose nanowhiskers or other rod-like particles, or the fines formed in conventional cellulose pulping processes, for example as small amounts on the surface of cellulose fibers.

Nanocrystalline cellulose or cellulose nanocrystals may be also suitable for use in the present bioreactor and methods. Nanocrystalline cellulose may be used in higher concentration compared to nanofibrillar cellulose, such as at a concentration in the range of 2-8% (w/w), such as 2-6% (w/w). Nanofibrillar cellulose and nanocrystalline cellulose may have different properties and may provide different functionalities in the present uses. Therefore it may be desired to use one of these materials for certain specific applications. The properties of both materials may be controlled and adjusted during manufacture, which enable preparing desired material for a specific purpose.

In one embodiment the nanostructured cellulose comprises or is nanocrystalline cellulose. Nanocrystalline cellulose may have a number average fibril diameter in the range of 2-40 nm, such as 2-20 nm, and a number average fibril length of 100 nm or more, up to several micrometres, such as in the range of 100-400 nm. Usually 10% or less of the material has a particle size of less than 5 µm. Nanocrystalline cellulose may be produced from cellulose by acid hydrolysis, which removes the amorphous region to obtain the crystalline region of cellulose. Therefore nanocrystalline cellulose practically consists of crystalline cellulose, and it lacks amorphous regions of cellulose. Nanofibrillar cellulose on the other hand includes both crystalline parts as straight segments and amorphous parts, which provide a kink in the fibril. Even though the both nanostructured cellulose materials have common features and properties, also because of the structural differences the two materials may also exhibit some different properties.

The embodiments and examples disclosed herein referring to nanofibrillar cellulose may be also applied to nanocrystalline cellulose. For example the hydrogels or entities may be also formed from nanocrystalline cellulose.

### Nanostructured cellulose entities

The "entities" as discussed herein, such as "NFC entities", "separate entities", "separate NFC entities" or the like expressions refer to any suitable forms formed from the nanostructured cellulose discussed herein. Such entities may be present or called as bodies or hydrogel bodies. The entities may be provided in a suspension. The hydrogel may be in a variety of forms, such as continuous, partly or fully discontinuous, such as globular or elongated forms, for example including a plurality of beads or the like entities in discontinuous form, which entities may be separate or (partly) interconnected. However, the structure of the entities is homogenous. The entities may also be fibers or filaments formed from nanofibrillar or nanocrystalline cellulose, so also these forms exhibit the properties of nanofibrillar or nanocrystalline cellulose hydrogel and shall not be confused with cellulose fibers or fibers or filaments of other materials. The entities may be called as three-dimensional entities, which is intended to distinguish from thin layers of nanostructured cellulose.

The entities may have an average (smallest) diameter of 100 µm or more, such as an average smallest diameter in the range of 100-5000 µm. The highest average diameters may be present when the entities have elongated shape, such as fibers of filaments, which may have an average smallest diameter (e.g. width) in the range of 200-5000 µm, such as 500-3000 µm. The length of such elongated entities may be 1 mm or more, even centimeters, such as 1-10000 mm. Entities having a spherical or globular form, such as beads, granules and the like may have a lower average diameter, such as 100-500 µm, for example 150-350 µm.

Providing the nanostructured cellulose in the form of such entities help controlling the conditions of the cell culture. For example the viscosity of a suspension of separate nanostructured cellulose entities is much lower than a suspension of homogenous nanostructured cellulose, especially nanofibrillar cellulose, which in most cases is present as a viscous hydrogel. This makes mixing of such viscous suspension challenging, especially when the concentration increases. The separate entities on the other hand may each have a relatively high nanostructured cellulose concentration, fibrillation degree and/or aspect ratio, but still they can be handled without problems. The same may apply also for properties such as migration of substances, such as nutrients, gases, metabolites and/or cell-derived products, which may be more easily released from the separate entities compared to homogenous nanostructured cellulose hydrogel with a large volume. Separate entities also enable culturing the cells on a surface and/or below the surface of the entities, i.e. embedded, which may be desired in some cases. For example the exchange of substances, including release of the cell-derived products, may be faster and/or more efficient in such cases. It is also possible to better maintain the structure of produced and released extracellular vesicles and the like cell-derived structures, especially ones having a lipid monolayer or bilayer. It was also noted that using such entities may be desired for cells incubated, cultured and/or provided at a stressed state. A high stress threshold can be maintained in such case. When cells are incubated or cultured on and/or in the entities, especially inside the entities, the cells can tolerate higher flow and stronger mixing or agitation. This enables providing more efficient perfusion reactor and conditions, more efficient mixing, and more efficient production of cell-derived products.

One example provides a nanostructured cellulose entity for culturing cells comprising an aqueous medium and hydrogel bodies comprising the nanostructured cellulose product suspended in the aqueous medium. The nanostructured cellulose entity may be applied to and/or it may be in the first compartment of the bioreactor. The aqueous medium may be cell culture medium. In one example the nanostructure cellulose entities are interconnected. In one example the nanostructure cellulose entities are not interconnected. The nanostructure cellulose entities may be provided at a water content in the range of 1-90% (w/w), more particularly 40-99% (w/w) or 90-99% (w/w). In general the water content is still high in such hydrogel bodies or beads. They are present in a form of hydrogel clumpses or swollen microbeads rather than solid cellulose particles. Even spray dried particles need to swell considerably in bioreactor to be cell compatible in properly.

The nanostructured cellulose entities are obtainable by a method comprising steps of providing the nanostructured cellulose product in a first aqueous medium to provide a hydrogel, and mixing said hydrogel with a second aqueous medium to obtain a suspension of hydrogel bodies in the second aqueous medium. The first and the second aqueous medium can be same or different, wherein they may be of same medium type, but they may also be different, for example the first medium being e.g. cell storage medium and the second medium being cell culture medium. The nanostructured cellulose entities can be made also from concentrated nanostructured cellulose hydrogels or from dry nanostructured cellulose by granulating the concentrated hydrogel or dry nanostructured cellulose to obtain granules, hydrating the granules in an aqueous medium, and mixing the hydrated granules, optionally adding aqueous medium, to obtain a suspension of hydrogel bodies. The discontinuous structure of the nanostructured cellulose entity suspension can be verified e.g. by simple microscopic analysis or viscosity and/or yield stress determination and comparison with a continuous homogeneous hydrogel having the corresponding nanostructured cellulose concentration. The yield stress of the nanostructured cellulose entity suspension is lower than the yield stress of the corresponding continuous homogeneous hydrogel at the same conditions, such as 1-95% of the yield stress of the corresponding continuous homogeneous hydrogel at the same conditions. The same applies to viscosity, which makes handling of the nanostructured cellulose entity suspension easier.

Discontinuous gel structures can be made also from concentrated (e.g. 10-30% w/w) or even from dry nanostructured cellulose products. When using dry or concentrated materials, the sample is first granulated to an appropriate size, for example an average diameter of 0.1-2 mm, hydrated in water or in cell culture medium, and then activated into either continuous or discontinuous form using appropriate methods. Spray dried particles, having an average diameter in the range of 2-20 micrometers, can be also used as a starting material. The controlled porosity in these kinds of discontinuous gels is dependent on particle size and the total concentration, *i.e*. distance between the swollen gel domains or gel bodies

In one example the total volume of the hydrogel bodies from total volume of the nanostructured cellulose entity suspension is in the range of 10-99% (v/v), such as 50-95% (v/v).

One example provides a discontinuous nanostructured cellulose entity suspension and a method for producing thereof, wherein the method comprises
- providing nanostructured cellulose product in a form of
   i) a homogeneous hydrogel;
   ii) a combination of the homogeneous hydrogel with an aqueous medium; and/or
   iii) dehydrated gel bodies or dry granulated nanostructured cellulose product hydrated in an aqueous medium; and
- mixing at conditions favouring mechanical disruption of the homogeneous structure of the hydrogel to obtain a suspension of nanostructured cellulose entities, for example hydrogel bodies as a three-dimensional discontinuous entity.

The fraction volume of the gel bodies comprising the three-dimensional discontinuous entity may be in the range of 50-99% of the total volume of the three-dimensional discontinuous entity and, accordingly, the local nanostructured cellulose concentration may be higher or lower than that of the total entity. The fraction of the gel bodies may be qualitatively determined readily e.g. by inspection under microscope or by sedimentation analysis.

The term "three-dimensional discontinuous entity" refers to a system having three-dimensionally discontinuous structure. Said entity comprises an aqueous medium and hydrogel bodies comprising cellulose nanofibrils and/or derivatives thereof suspended in the aqueous medium. The term "three-dimensional" is used to distinguish from "two dimensional", which refers to a system based on a layer, such as cell culture in a layer, which has a low thickness. Three dimensional systems may be established in the reactor discussed herein.

"Discontinuous" refers to the heterogeneous structure of the entity or to interruptions in the physical continuity within the entity, for example interruptions in the aqueous medium by hydrogel bodies or interruptions in and/or between hydrogel bodies by the aqueous medium. In general the discontinuous material may comprise a plurality of separate, including partly separate, bodies, domains, granules, particles and the like, which may have substantially spherical, elliptical, elongated, or the like, or uneven shape. The plurality of bodies, domains, granules, particles and the like may be also partly interconnected in the discontinuous material. Discontinuous refers to material which is not substantially homogenous. For example a block or a membrane of hydrogel is not discontinuous, but plurality of beads, spheres or the like separate bodies suspended in liquid medium form a discontinuous entity, even if some of the bodies are attached to each other. In one embodiment the nanostructured cellulose is in a form of separate bodies, which may be hydrogel bodies, such as beads.

"A hydrogel body" and "a hydrogel domain" refer to an aliquot, a division, a domain, a fraction, a portion or a dose of a hydrogel, preferably having a continuous inner structure. The hydrogel body may have a well-defined, indefinite, symmetrical or asymmetrical shape. The "entity" as used herein may refer to such a hydrogel body or a domain.

"Suspended" or "suspension" when used in context of three-dimensional discontinuous entity or hydrogel bodies refers to a heterogeneous mixture of an aqueous medium and hydrogel wherein the hydrogel may be present as separate and/or interconnected hydrogel entities.

"Interconnected" and "interconnection" when used in context of hydrogel entities refers to a system where the hydrogel entities are in contact with each other. The contact may be a direct connection between the hydrogel entities or the hydrogel entities may be loosely connected. When the homogeneous structure of the hydrogel is broken e.g. by mixing, the resulting discontinuous structure may be characterized by hydrogel entities of different sizes and forms. The resulting system may contain aqueous cavities between interconnected hydrogel entities or the loosely connected hydrogel entities may "float" in the aqueous medium having contacts with each other. The hydrogel entities may be indirectly connected via e.g. cells or other components present in the system.

"Dehydrated" or "dewatered" form refers to form of the material in which some but not necessarily all water is removed from the material in question. Thus, the term dehydrated encompasses e.g. concentrated slurries, granules, flakes, and powders. The dehydrated material may have a water content in the range of 0-90% (w/w), such as 0-80% (w/w), 0-50% (w/w), 1-50% (w/w), 1-40% (w/w), 1-30% (w/w), 1-20% (w/w), 10-50% (w/w), 10-40% (w/w), 10-30% (w/w), or 1-10% (w/w).

### Culturing methods

The extraction, culturing or cultivation discussed herein refer to a process wherein the cell-derived products such as biologics are being produced by the cells incubated and/or cultured in the nanostructured cellulose hydrogel material, and wherein the cell-derived products are subsequently released from the nanostructured cellulose hydrogel for collection as biologically active product, *i.e*. in an active form.

The bioreactor, including the materials contained therein, may be used in a method for separating cell-derived products from cultured and/or incubated cells. The method may be also a method for culturing and/or incubating cells, and/or a method for producing cell-derived products.

The present application also discloses use of the bioreactor for separating cell-derived products from cultured cells. The present application also discloses use of the bioreactor for culturing cells. The present application also discloses use of the bioreactor for producing cell-derived products.

The present application discloses use of nanostructured cellulose, especially as an hydrogel having an average pore size in the range of 100-1000 nm or a median pore size in the range of 0.1-10 µm², such as 0.3-2.0 µm², for extracting cell-derived products from incubated cells with any of the methods disclosed herein. The present application discloses use of nanostructured cellulose having a median pore size in the range of 0.1-10 µm², such as 0.3-2.0 µm², for extracting cell-derived products from incubated cells with any of the methods disclosed herein. In one embodiment the nanostructured cellulose is nanofibrillar cellulose. In one embodiment the nanofibrillar cellulose is native nanofibrillar cellulose, such as chemically unmodified nanofibrillar cellulose, preferably also enzymatically and/or physically unmodified nanofibrillar cellulose. The median pore size is preferably present and/or detectable from nanostructured cellulose hydrogel in the concentration of use, which may be in the range of 0.1-8% (w/w), such as 0.2-6% (w/w) or in a subrange, as described herein.

The present disclosure provides a nanostructured cellulose product for extracting cell-derived products from cultured cells, the product comprising native nanostructured cellulose hydrogel having a median pore size in the range of 0.1-10 µm², such as 0.3-2.0 µm². The native nanostructured cellulose is chemically unmodified nanostructured cellulose, but it may further be enzymatically unmodified and/or physically unmodified nanostructured cellulose. Chemically unmodified nanofibrillar cellulose may be desired for applications involving recovery of cell products, as chemically modified nanofibrillar cellulose could contain such additional chemical groups which would change the properties of the nanofibrillar cellulose and prevent or slow the release and migration of cell-derived products. For example the added chemical groups may bind the cell-derived products and/or the fibril diameter may be so low that the rheological properties and/or the porosity of the hydrogel would be less suitable for the present use compared to chemically unmodified nanofibrillar cellulose. Chemically and enzymatically unmodified nanofibrillar cellulose also maintains the original fibril length, which has an impact to the properties of the material.

The product may be provided as a packed product, for example packed in a vial, a tube, a container, a syringe, a bottle, a flask or any other applicable container, usually a sealed container. The material may be provided in a concentration which is the concentration during usage, or a concentration which is suitable for adding to the bioreactor, such as any concentration disclosed herein. The product may therefore be a ready-to-use product. In one embodiment the nanostructured cellulose is nanofibrillar cellulose.

The median pore size, which is herein presented as a pore area, may be determined microscopically, such as from microscopic images, for example SEM images. Median pore size describes the size of the useful pores in the hydrogel better than an average pore size as the hydrogel also contains large cavities, which would raise the average pore size value. The pore size may be derived from (internal) pore width, which may be the diameter of a cylindrical pore or the distance between the opposite walls of a slit, which is a representative value of various sizes of vacant space inside a porous material. A pore diameter may be a diameter of a pore in a model in which the pores typically are assumed to be cylindrical in shape and which may be determined or calculated from data obtained by a specified procedure. It may be a median pore diameter, which is a diameter that corresponds to the 50th percentile of pore volume, i.e. the diameter for which one half of the pore volume is found to be in larger pores and one half is found to be in smaller pores.

The nanostructured cellulose may be in any of the forms disclosed herein, such as in (homogenous) hydrogel or as (separate) entities. The cells and the cell-derived products may be any of the ones disclosed herein.

The method may comprise
- providing the bioreactor,
- providing cell culture medium, which is preferably serum-free, animal origin free, feeder-free and/or xeno-free cell culture medium,
- providing cells,
- culturing and/or incubating the cells in the compartment comprising nanostructured cellulose and cells to form cell-derived products,
- optionally mixing the nanostructured cellulose and cells,
- allowing cell-derived products to diffuse from the cells, such as from the compartment comprising nanostructured cellulose and cells, into the cell culture medium,
- harvesting the cell culture medium comprising cell-derived products from the bioreactor, preferably via the outlet for extracting cell culture medium comprising cell-derived products, to separate cell-derived products from the cultured cells.

The cell culture medium may be any applicable cell culture medium, which can be used in the bioreactor disclosed herein. Usually the cell culture medium is aqueous medium, such as aqueous solution or dispersion, or such aqueous medium is provided to form the cell culture medium, which may also include the nanostructured cellulose, such as in gel form. Cell culture medium may comprise an appropriate source of energy and compounds which regulate the cell cycle. A culture medium may comprise a complement of amino acids, vitamins, inorganic salts, glucose, and may contain serum as a source of growth factors, hormones, and attachment factors. In addition to nutrients, the medium also helps maintain pH and osmolality.

Preferably the cell culture medium contains no or only traces of other type of cell-derived material. A "trace" may refer to less than 0.5% (w/w), less than 0.1% (w/w), less than 0.05% (w/w) or less than 0.01% (w/w), or to amounts which cannot be detected using common methods for detecting or identifying specific biological compounds, such as immunological methods. The cell culture medium is preferably serum-free, animal origin free, human origin free, blood origin free, feeder-free and/or xeno-free cell culture medium. Feeder-free refers to cell culture medium and/or conditions wherein feeder cells are not present, for example wherein cells are cultured in absence of feeder cell layer. Such medium preferably contains only the essential cell culture compounds needed for desired cell culture, such as stem cell culture. It is also possible that during the cell culturing the culture medium may be changed into such medium in a last step, i.e. before allowing cell-derived products to diffuse from the cells.

Incubating may also refer to culturing and/or maintaining the cells, and it is generally carried out for a time period sufficient to obtain the cell-derived products from the cells. In general "culturing" as used herein refers to the incubating. The cells may be incubated at conditions allowing the cells to produce the cell-derived products, or at conditions facilitating the production of selected or desired cell-derived product(s). The conditions may include for example suitable time, suitable type of cell culture medium, suitable type and/or concentration of the nanostructured cellulose, and/or suitable temperature. It is also possible to add one or more substance(s) facilitating the production of the desired cell-derived product(s), such as to add the substance(s) to the cell culture medium.

Production of cell-derived products, such as EVs, from cells incubated or cultured in a bioreactor type of culture system can be performed in the different ways, which may utilize mixing, such as stirring. The bioreactor may be divided into compartments for example by using a structure such as a membrane, such as a semi-permeable membrane, which is permeable to cell culture medium comprising the cell-derived products, and impermeable to cells and nanostructured cellulose. The cells may be in a first compartment, which may be called as static compartment and/or wherein the cells are substantially fixed, and a flow of culture medium is arranged on and/or through the other side of the membrane, preferably at and/or to a second compartment, or at and/or to a further compartment. The cell-derived products may be harvested from the second compartment and/or from a further compartment. Such bioreactor may be implemented in various sizes, for example by using cell culture wells equipped with a membrane insert, for example products such as Transwell cell culture inserts, by using cell culture flasks or bottles, or by using large vessels.

The method may comprise providing nanostructured cellulose, such as in any suitable form discussed herein, providing cells, such as any suitable cells discussed herein, and combining the nanostructured cellulose with the cells. The nanostructured cellulose and the cells may be combined before applying the combination or mixture into the reactor, or the nanostructured cellulose may be applied into the reactor first, and the cells may be applied to the reactor subsequently. This may be carried out batchwise or continuously, in respect of the nanostructured cellulose or the cells, or both. The nanostructured cellulose and the cells may be provided as a suspension.

The method may comprise embedding the cells, or allowing the cells to embed, in the nanostructured cellulose entities. This may include allowing the cells to enter the entities. This may be controlled by providing the entities in a form and/or composition allowing the cells to enter, such as at a suitable concentration, comprising nanostructured cellulose having a fibrillation degree and/or chemical composition or other properties allowing this. For example a lower concentration of the nanostructured cellulose, and/or lower fibrillation degree and/or lower aspect ratio may be used to obtain entities or hydrogel which is more permeable to the cells. In a similar way higher concentration, fibrillation degree and/or aspect ratio may be used to prevent or restrict the cells to enter the entities or hydrogel, so the cells may be cultured on top/surfaces of the entities or hydrogel and/or immediately below the surfaces of the entities. In such case the method may comprise attaching the cells, or allowing the cells to attach, to the surface of the nanostructured cellulose entities and/or encapsulating or embedding the cells inside the nanostructured cellulose.

The nanostructured cellulose may be provided with a suitable porosity degree, which facilitates embedding the cells into the material, and also exchange of substances, such as liquid flow and/or migration of the cell-derived products, especially in vesicle form. The porosity especially allows encapsulating the cells inside the nanostructured cellulose. The average pore size may be a number-average pore size. The porosity can be also presented as a pore area, which may be median pore size. In one embodiment the nanostructured cellulose has a median pore size in the range of 0.1-10 µm², such as 0.3-2.0 µm². This can be obtained by providing nanostructured cellulose with suitable fibril size and aspect ratio, fibrillation degree, modification degree, and/or concentration of the hydrogel. It was found out that with the porosity of the nanostructured cellulose hydrogel at said range the cells were efficiently immobilized in the nanostructured cellulose matrix but the cell-derived products were easily released from the cells and the hydrogel and could be separated and recovered without problems. The structure of vesicle type of cell-derived products and the type of cell-derived products in general were not altered. Also the cells maintained their desired phenotype and could tolerate mechanical stress during the incubation, such as mixing and/or flow.

The nanostructured cellulose entities may be any entities discussed herein, such as beads or the like, for example microbeads. Most nanostructured cellulose entities may have a volume median particle size in the range of 10-1000 µm. Microbeads or other similar globular or spherical entities may have volume median particle size in the range of 50-500 µm. Such beads and the like may be formed by using microfluidics system, electro spraying or printing or dispensing.

By using the nanostructured cellulose and the bioreactor discussed herein it is possible to implement the method as a continuous or semi-continuous method, so it is possible to maintain the production of cell-derived products for a relatively long period of time, such as for weeks or even for months. The production rate can be increased and maintained at high level without substantial variation in the production rate or in the quality of the cell-derived products. The cells can be maintained in a desired state, such as exhibiting a desired phenotype, and the type(s) of cell-derived product(s) can be maintained so that same kind of cell-derived products can be obtained over the whole production period.

In one embodiment the method is a continuous method, which may include a semi-continuous method wherein cell-derived products are harvested batchwise and new medium is added at regular intervals. However especially in a fully continuous method it is not necessary to allow the cells or the cell carrier or matrix to settle, or to separate it from the medium, or stop flow, mixing or agitation, before harvesting the cell-derived products. It is not necessary to change temperature or other conditions of the system or the culture. Therefore the cells are not endangered or disturbed during the process as they can continuously receive oxygen and nutrients and are not packed or otherwise subjected to additional mechanical forces or to other sudden changes in the environment. The production of cell-derived products is not stopped or slowed down during a continuous production, so the efficiency of the process is maintained as well as the constant quality of the produced products.

In one embodiment the method comprises adding new cell culture medium to the bioreactor at an equal rate to the rate which the cell culture medium comprising cell-derived products is harvested from the bioreactor.

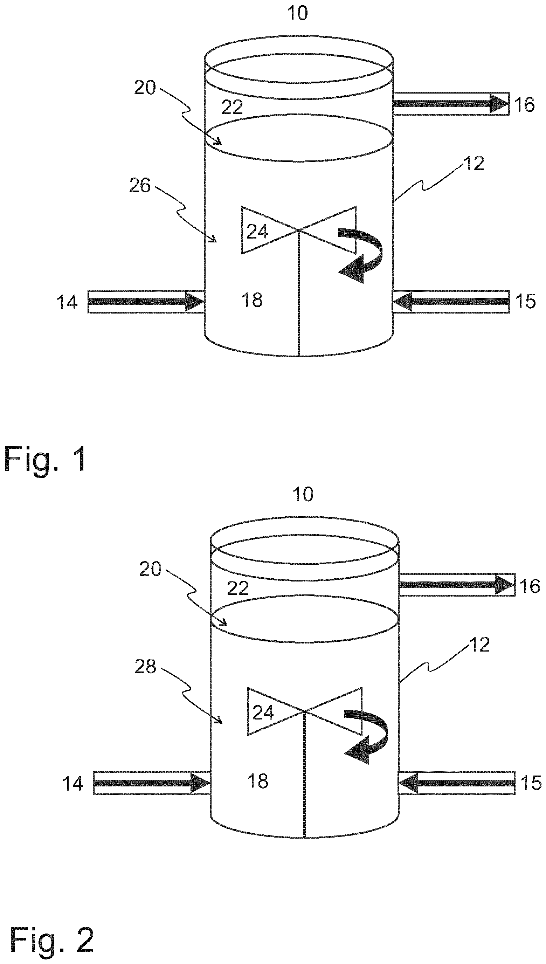

Figure 1 presents an example of the bioreactor 10 setup comprising a container 12, which is divided into a first compartment 18 and a second compartment 22 with the first surface 20 comprising a permeable membrane having a pore size in the range of 0.4-3 µm. The first compartment 18 contains a dispersion of nanostructured cellulose and cells 26 and is equipped with a mixer 24. The dispersion of nanostructured cellulose and cells 26 may be conveyed into the first compartment via a first inlet 14, and cell culture medium may be conveyed into the first compartment via a second inlet 15. The cell-derived product rich culture medium is separated via the membrane at the surface 20 into the second compartment 22 and can be conveyed out from the container via the outlet 16.

In one example the cells are mixed within nanostructured cellulose to obtain a mixture, which may be at a desired concentration. The mixture, i.e. the whole culture system, is mixed or stirred, for example with a mixer or a stirrer such as one comprising a rudder, to facilitate and/or maintain homogenous mixing. It is also possible to obtain the mixing without using or providing a mixer or a stirrer.

In one example the method is carried out as a batch method, such as a batch production of cells in a short time of for example 12-72 hours. In such case stirring may not be necessary. In one example a bioreactor connected to a rocking base, such as WAVE bioreactor, is used. Such system is not stirred and does not contain a stirrer, but the mixing is obtained by moving in a rocking manner the bioreactor, which may comprise a vessel or container, such as a bag, containing the cell culture and nanostructured cellulose. The rocking motion induces waves in the cell culture medium to provide mixing and oxygen transfer.

The cells may be mixed with nanostructured cellulose hydrogel or dispersion, so in such an option the nanostructured cellulose is not present as separate entities. The cells and nanostructured cellulose are mixed and formed into a mixture in the cell culture medium, more particularly a homogenous mixture or substantially homogenous mixture. The mixture may be in a form of a hydrogel. The cells may be cultured within nanostructured cellulose without specification of cellular/nanostructured cellulose interactions or morphology.

In one embodiment the cells are embedded in the nanostructured cellulose entities and/or attached to the surface of the nanostructured cellulose entities. Using such entities and combining the cells to the entities in a selected manner facilitates and helps controlling cell culturing, cell incubating, cell-derived product formation and/or release, exchange of nutrients, liquids and/or gases, and the like properties. It is possible to use a structure or other means between the first and the second compartment with higher pore size to facilitate the flow of medium as the entities cannot pass the pores. By using entities the mixing of the mixture of cells and nanostructured cellulose can be facilitated as the suspension of entities has a relatively low viscosity.

Figure 2 presents an example of the bioreactor 10 setup comprising a container 12, which is divided into a first compartment 18 and a second compartment 22 with the first surface 20 comprising a permeable membrane having a pore size in the range of 0.4-3 µm. The first compartment 18 contains a suspension of nanostructured cellulose entities and cells 28 and is equipped with a mixer 24. The suspension of nanostructured cellulose entities and cells 28 may be conveyed into the first compartment via a first inlet 14, and cell culture medium may be conveyed into the first compartment via a second inlet 15. The cell-derived product rich culture medium is separated via the membrane at the surface 20 into the second compartment 22 and can be conveyed out from the container via the outlet 16.

In one example a process of production of extracellular vesicles (EV) is as follows. Nanostructured cellulose is mixed with culture medium and cells to final concentration to obtain a mixture of cells and nanostructured cellulose. The mixture of cells and nanostructured cellulose is added to a bioreactor. The mixture of cells and nanostructured cellulose will either be maintained by the addition of new media during culture or removed, such as siphoned off, and replaced with fresh cells and/or nanostructured cellulose. The cells are allowed to produce EVs and secrete them to the medium. New medium may be added to the system as EV-enriched medium is removed. EV-enriched medium may be harvested at a rate equal to addition of new medium, where a permeabilized membrane will separate cells and nanostructured cellulose from the EV-enriched medium.

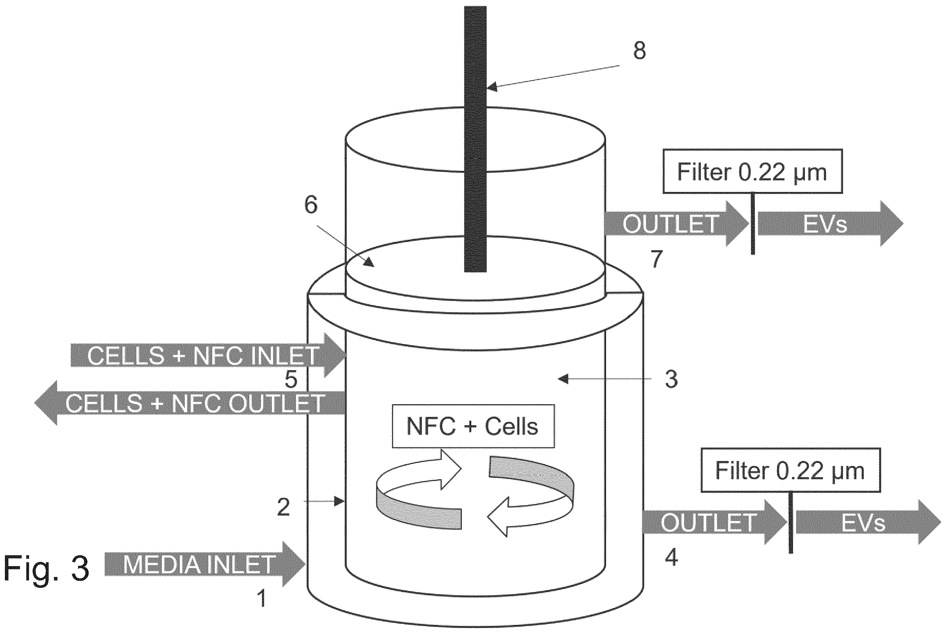

Figure 3 presents an example of the bioreactor setup comprising a media inlet 1 for new media to feed the cells into a container and an outlet 4 for used media to be exchanged, as well as to isolate EVs or other cell-derived products from the culture. The container includes a permeable membrane 2 which will only allow the media to diffuse in and out to the cells to feed the cells, such as for allowing dissipation of media into a culture chamber. Some EVs may be able to come out through the membrane here, but these can be collected through the outlet port 4. Cells mixed in with NFC 3 are in a compartment/culture chamber, however also nanocrystalline cellulose may be applied instead. The NFC may be for example 0.5% (w/w) continuous hydrogel or microbeads. An inlet and/or an outlet 5 may be present allowing for the exchange of cells embedded in NFC should the application require it. Separating permeable membrane or mesh 6 allows filtration of cell products from cells. The membrane of mesh may have a pore size in the range of 0.4-3.0 µm. The bioreactor comprises an outlet 7 of cell products for filtration for isolation of EVs. Plunger 8 can be used if the application requires it, to compress the cells with NFC. The outlets 4 and 7 comprise a filter with a cut-off size of 0.22 µm. Therefore this example contains two outlets, wherein the lower outlet 4 may be used primarily for exchanging the medium, and the upper inlet may be used primarily for separating and recovering the cell-derived products.

In one embodiment the nanostructured cellulose is in the form of separate entities, such as hydrogel beads, porous beads, fibers, filaments and/or a membrane. This enables controlling the nanostructured cellulose in the reactor, and it may also facilitate the exchange of gas and/or liquid to the cells. Further, it is possible to further control the cultivation, as the cells may be cultured on top or on the surface of the entities, or they may be allowed to penetrate and embed the entities. Mixing of the culture in the reactor may be easier and/or more efficient.

In another example the cells are seeded on top of nanostructured cellulose entities and cultured as adherent cells, and/or the cells are embedded within nanostructured cellulose entities. The entities may be beads or the like substantially globular forms, or elongated entities such as fibers or filamens. The mixture, i.e. the whole culture system, is mixed or stirred, for example with a mixer or a stirrer, such as one comprising a rudder, to facilitate and/or maintain homogenous mixing. It is also possible to obtain the mixing without using or providing a mixer or a stirrer, as discussed in previous.

In one example the process of production of cells on top of entities, such as beads, is as follows. Nanostructured cellulose is provided as entities or made into entities, such as microbeads, mixed with culture medium and cells to a final concentration obtain a mixture of cells and nanostructured cellulose entities. The mixture of cells and nanostructured cellulose entities is added to a bioreactor. The cells are allowed to produce EVs and secrete them to the medium. The mixture of cells and nanostructured cellulose entities is either maintained by the addition of new medium during culture, or removed, such as siphoned off, and replaced with fresh cells and/or nanostructured cellulose entities. New medium may be added to the system as EV-enriched medium is removed. EV-enriched medium may be harvested at a rate equal to addition of new medium. A permeabilized or semi-permeable membrane may be used to separate cells and NFC entities from the EV-enriched medium.

In one example the process of production of cells embedded within nanostructured cellulose is as follows. Nanostructured cellulose is mixed with medium and cells, and then formed into entities such as microbeads, or alternatively into other forms, for example using extrusion methods. The mixture of cells and nanostructured cellulose entities is added to bioreactor. The cells are allowed to produce EVs and secrete them to the medium. The mixture of cells and nanostructured cellulose entities is either maintained by the addition of new medium during culture, or removed, such as siphoned off, and replaced with fresh cells and/or nanostructured cellulose entities. New medium may be added to the system as EV-enriched medium is removed. EV-enriched medium may be harvested at a rate equal to addition of new medium. A permeabilized or semi-permeable membrane may be used to separate cells and nanostructured cellulose entities from the EV-enriched medium. Alternatively, the bioreactor may be a tank of nanostructured cellulose /media with beads or gel which is based on top of a rocker to create mixing.

In one example the bioreactor is implemented with a container comprising adjustable volume, such as a bellow bottle, wherein the container comprises a part which can be mechanically manipulated to change the volume of the part (the bellow). The container may be made or comprise a part made of elastic material, such as plastic, elastomer or the like. The cells and nanostructured cellulose, such as nanostructured cellulose entities, in cell culture medium are placed in the container, and the volume of the container is changed to create a flow of the medium in the container and mix the content of the container. An external actuator may be provided and arranged to manipulate the adjustable volume part of the container, for example automatically and/or controlled by a control unit. The actuator may be connected to movable part in the container or a compartment, such as a piston, a plunger or the like or a deformable part of the container or a compartment, such as a bellow or the like. An example of such arrangement provides automated extracellular vesicle production using NFC hydrogel fibers, which are formed by combining isolated nanostructured cellulose fibrils, in a bellow bottle comprising a controller and a mechanical device for operating the bellow bottle, which is made of elastic material. When the bellow is stretched or released to obtain the largest volume of the container, the bellow is filled with the cell culture medium, but the nylon mesh above the bellow prevents the cells and nanostructured cellulose fibers to enter the bellow part. When the bellow is pressed, the liquid enters from the bellow to the upper compartment and the content therein is mixed. It is easy to separate the EV-rich medium from the nanostructured cellulose fibers comprising the cells when desired.

The means for mixing may therefore comprise a mixer, which may be a mechanical mixer, which may be fully or partly in a compartment, or the means for mixing may comprise means for providing flow of medium, or/such as means for changing, altering or otherwise adjusting the volume of the container or an compartment, such as repeatedly increasing and decreasing the volume, i.e. in a pulsed manner, as discussed in previous. When such mixing means are used that do not include contact of the mixing means, such as a mixer, with the cells and/or nanostructured cellulose, the cells and/or cell-derived products can be protected from mechanical stress during the process.

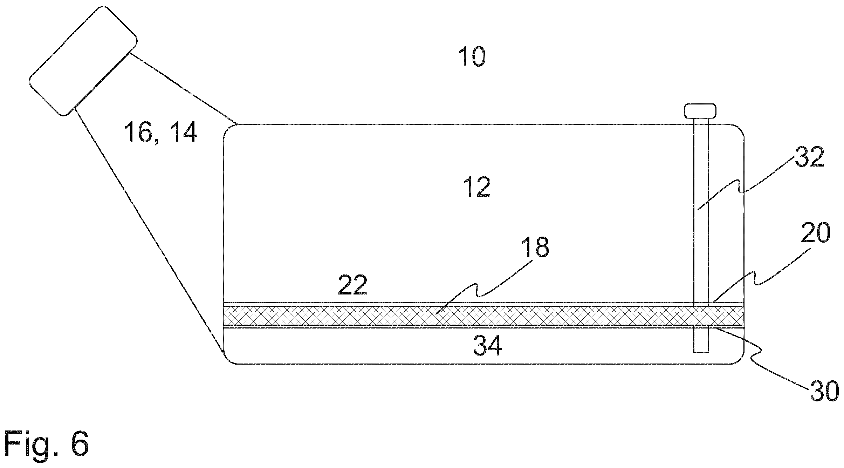

In one example the bioreactor is implemented in a flask, such as a cell culture flask such as shown in Figure 6. The flask may be divided into compartments with one or more membranes 20, 30, such as with a semi-permeable membrane with a suitable cut-off size for nutrient and cell-derived product exchange 20, which membrane is above the cell compartment 18 (first compartment), during the incubation, and separates the cell culture compartment (the first compartment) from the second compartment 22, which acts as a cell-derived product compartment. There may be also a further surface 30, membrane or the like structure for gas exchange, which may be at a different side of the first compartment 18 from the first surface, such as below the cell compartment during the incubation, for example the silicone membrane 30 of Figure 6. Also in this type of bioreactor both nanostructured cellulose hydrogel and separate nanostructured cellulose entities may be used. The gas exchange may be facilitated by providing a gas compartment (a third compartment) 34, which is separated by the cell compartment 18 by said second membrane 30, and which may act as oxygen support chamber and which may for example comprise support material with gas ducts. Figure 6 shows also a gas exchange tube 32 which is arranged between the outside of the flask and the gas compartment 34. The tube 32 or a similar tube may be also used as an inlet for the medium, if necessary, for example when such an inlet tube is arrange above the first compartment or into the first compartment. The medium may be alternatively or in addition be inputted from the mouth of the flask which may therefore act as an inlet 14 and/or an outlet 16. The flask may be a shake flask, and the method may include shaking the flask. However, it is possible to arrange a flow and/or mixing of cell culture medium to the flask with other means and methods, such as discussed herein, for example by providing a flow of liquid through the inlet and outlet.

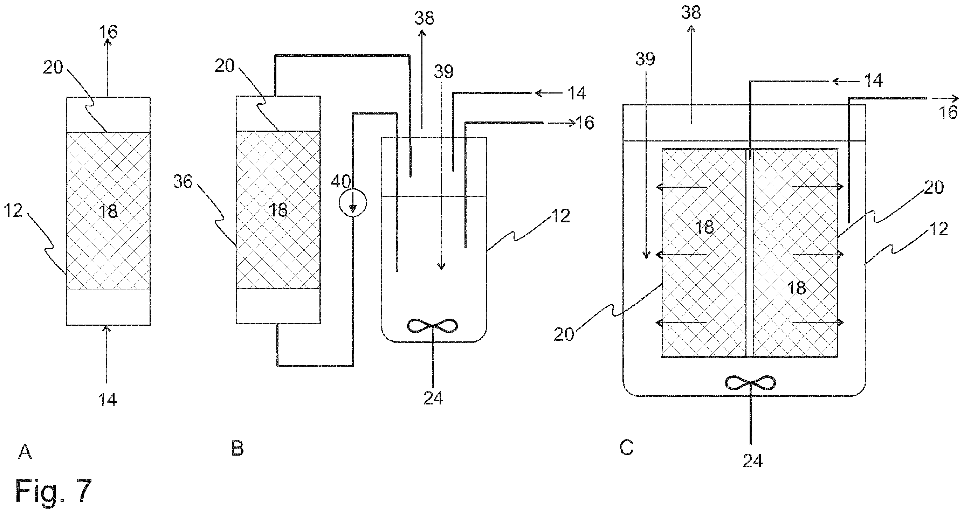

The compartment comprising nanostructured cellulose and cells may be a separate unit to the container. Such a separate unit may be or comprise another container, *i.e*. a second container, which may be inside the first container, or which may be outside the first container. Such a second container may be for example a fixed bed reactor, such as shown in Figure 7. Figure 7A shows a fixed bed type of arrangement, which comprises fixed bed material 18 for receiving and/or for culturing the cells. The fixed bed material 18 comprises the nanostructured cellulose, such as in the form of the separate entities, as disclosed herein. A flow of liquid, such as cell culture medium, is arranged through the fixed bed from the inlet 14 to the outlet 16, so the it is possible to provide feed of the medium and harvest of the cell-derived products, as shown in Figure 7A. The fixed bed may be provided in a container 12 or in a second container 36 (Figure 7B). The container may contain an inlet (feed) and an outlet (harvest) for the liquid either directly and/or connected to another container 12. The second container 36 may comprise a first surface 20 for providing cell culture medium comprising cell-derived products from the cells to the outlet, which first surface may comprise a membrane or other applicable structure or surface. The second container 36 may also comprise one or more further membrane(s), mesh(es) or other structures for retaining the fixed bed and allowing liquids, gases and cell-derived products to pass.

The fixed bed 18 in a separate container 36 may be connected to a container 12, such as the container of the bioreactor and/or other container, which may act as a conditioning vessel, as shown in Figure 7B. The fixed bed is connected to the container with tubes, hoses or the like structures for conveying liquid or for allowing liquid flow. The cell culture medium may be circulated in the system with the pump 40 so that it flows through the fixed bed 18 and from the outlet of the fixed bed to the container 12, which is provided with a mixer 24 and wherein the medium is mixed. Gases may be exchanged in the container 12, so that waste air may be released 38 and new gases may be inputted 39. Also new culture medium may be fed into the container, and cell-derived products may be harvested from the container 12. The cell culture medium may be further circulated back to the inlet of the fixed bed.

Alternatively the fixed bed 18 may be inside the container 12, as shown in Figure 7C, which shows an arrangement wherein an inlet 14 is arranged into a fixed bead 18, which is permeable through a surface 20 to the surrounding medium in the container 12. An outlet 16 is arranged into the medium, which can be mixed with the mixer 24.

In one embodiment the bioreactor is a perfusion bioreactor. In such case the method may comprise perfusion cell culturing. Perfusion bioreactor enables continuous production of cell-derived products. Perfusion is upstream processing which retains cells inside the bioreactor while continually removing cell waste products and media depleted of nutrients by cell metabolism. Fresh media is provided to the cells at the same rate as the spent media is removed. One way to achieve perfusion is the use of hollow fiber filtration, but also other setups disclosed herein may be used, such as the fixed bed type of setup. The perfusion method may comprise providing flow of cell culture medium through the compartment comprising nanostructured cellulose and cells. Correspondingly the perfusion bioreactor may comprise means for providing flow of cell culture medium through the compartment comprising nanostructured cellulose and cells.

Nanostructured cellulose is especially suitable for perfusion type of cell culture methods, such as for perfusion-capable bioreactors, for example membrane-based bioreactors or other applicable perfusion-capable technologies. Perfusion supports cell culture over an extended period of time by continuously feeding the cells with fresh media and removing spent media while keeping cells in culture yielding concentrated lots of exosomes. Nanostructured cellulose hydrogel can be used to fill fixed-bed or hollow-fiber perfusion bioreactors for large-scale cell culture production, especially exosome production, as perfusion supports continuous culture and reduced serum/factor requirement.

It is possible to release the cells from the nanostructured cellulose by degrading the nanostructured cellulose enzymatically, such as by providing one or more cellulase enzyme(s) and allowing the nanostructured cellulose to degrade to release the cells. The cells and/or the degraded nanostructured cellulose may be separated and optionally harvested and/or recovered. This may be carried out for expanding the cell culture, or for collecting the cells for downstream processing, such as for analyzing and/or isolating RNA, DNA and/or protein.

### Examples

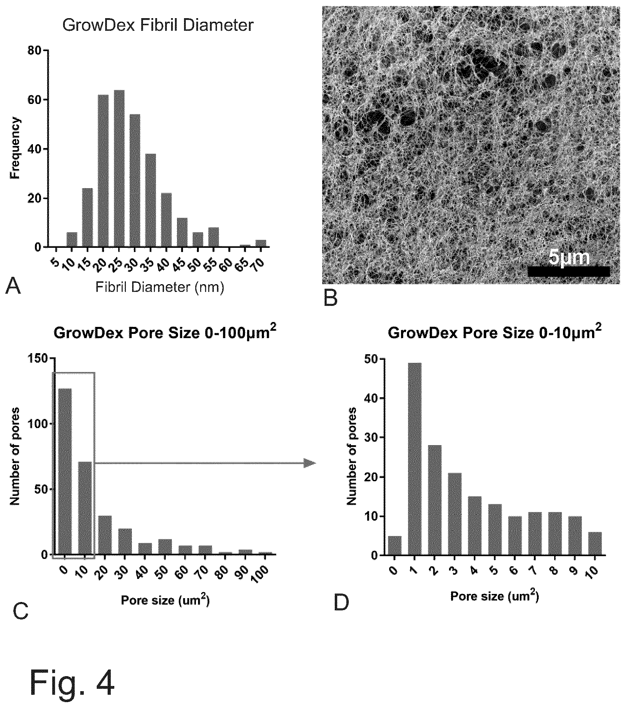

Different bioreactor setups as disclosed herein were tested. The fibril diameter of 0.5% (w/w) hydrogel derived from chemically native nanofibrillar cellulose from birch (GrowDex) were determined microscopically. Results from the most useful material for different applications, especially for extracting extracellular vesicles, are presented in Figure 4.

Figure 4A shows a fibril diameter distribution in the used GrowDex-derived nanofibrillar cellulose. Figure 4B shows a SEM image of a hydrogel wherein the pore sizes and shapes can be seen. Figure 4C and 4D present the pore size distribution of the hydrogel.

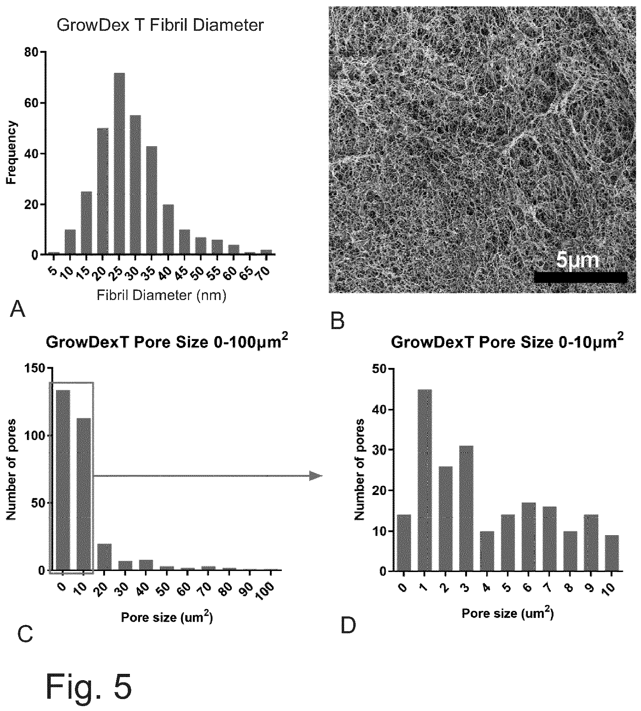

Similarly 0.5% (w/w) hydrogel derived from negatively charged nanofibrillar cellulose from birch (GrowDexT) was studied and the corresponding results are presented in Figures 5A-5D.

The scanning electron microscopy (SEM) was carried out with the following method. Hydrogels at 0.5 wt% (without or with cells) were fixed with 2% formaldehyde and 2% glutaraldehyde in 0.05M cacodylate buffer for at least 24 h before being rinsed. Hydrogels were washed twice with deionised water (DIW) to remove fixative and then carefully removed from their growth inserts by cutting the bottom mesh of the insert with a pointed razor blade. The samples were placed on 10 mm ø melinex coverslips (mesh-side down), excess water was gently removed, and the samples were then plunge-frozen in liquid nitrogen-cooled ethane. Subsequently, samples were freeze-dried overnight in an EMITECH K775X liquid-nitrogen cooled freeze dryer (Quorum Technologies). Melinex coverslips were mounted on aluminium SEM stubs using silver-DAG (TAAB); small amounts of silver-DAG around the bottom rim of the mesh/sample were used to secure the sample on the coverslip and ensure conductivity. Then, samples were coated with 35 nm gold and 15 nm iridium using an EMITECH K575X sputter coater (Quorum Technologies). Samples were viewed in a FEI Verios 460 scanning electron microscope at an accelerating voltage of 2 keV and a probe current of 50 pA. Images were acquired in secondary electron mode using either an Everhart-Thornley detector (ETD) or a Through-Lens detector (TLD) in immersion mode. The fibril diameters and pore sizes were determined from the images.

## Claims

1. A bioreactor (10) for extracting cell-derived products from cultured cells, the bioreactor comprising
- a container (12),
- an inlet (14) for inputting cell culture medium into the container,
- an outlet (16) for outputting cell culture medium comprising cell-derived products from the container,
- the container (12) comprising, or being connected to, a compartment (18) comprising nanostructured cellulose configured to receive cells, said compartment comprising a first separating surface (20) separating the nanostructured cellulose from the outlet and allowing cell culture medium comprising cell-derived products to pass through the first separating surface (20), and optionally comprising a further separating surface (30) for exchange of gas.

2. The bioreactor of claim 1, wherein the first separating surface (20) comprises or is in a form of a structure, such as a membrane or a mesh, permeable to cell culture medium comprising the cell-derived products, and impermeable to cells and nanostructured cellulose, such as a membrane having an average pore size in the range of 0.4-3 µm.

3. The bioreactor of claim 1 or 2, comprising means (24) for mixing the nanostructured cellulose and cells in the compartment (18), such as wherein the means for mixing comprises a mixer, means for providing flow of medium, and/or means for adjusting the volume of the container or an compartment.

4. The bioreactor of any of the preceding claims, comprising means for providing flow of cell culture medium via the inlet (14) and/or the outlet (16), preferably through the compartment (18) comprising nanostructured cellulose, for example to obtain a perfusion bioreactor.

5. The bioreactor of any of the preceding claims, wherein the compartment (18) comprising nanostructured cellulose is a separate unit (36) to the container (12), such as a fixed bed reactor.

6. The bioreactor of any of the preceding claims, wherein the first surface is in a form of hollow fiber(s).

7. The bioreactor of any of the preceding claims, wherein the nanostructured cellulose is in the form of separate entities, such as hydrogel beads and/or porous beads.

8. The bioreactor of any of the preceding claims, wherein the nanostructured cellulose comprises nanofibrillar cellulose comprising cellulose fibrils and/or fibril bundles having a number-average diameter of 200 nm or less and/or wherein the nanofibrillar cellulose, when dispersed in water, provides a zero shear viscosity in the range of 100-50000 Pa s, such as in the range of 300-8000 Pa s, and a yield stress in the range of 1-50 Pa, such as in the range of 2-15 Pa, determined by rotational rheometer at a consistency of 0.5% by weight in aqueous medium at 22±1°C.

9. The bioreactor of claim 8, wherein the nanofibrillar cellulose is chemically modified nanofibrillar cellulose or chemically unmodified nanofibrillar cellulose, preferably also enzymatically unmodified nanofibrillar cellulose.

10. The bioreactor of claim 8 or 9, wherein the nanofibrillar cellulose comprises cellulose fibrils and/or fibril bundles having a number-average diameter in the range of 2-50 nm, such as 2-20 nm, and wherein the nanofibrillar cellulose, when dispersed in water, provides a yield stress in the range of 1-50 Pa, such as in the range of 2-15 Pa, determined by rotational rheometer at a consistency of 0.5% by weight in aqueous medium at 22±1 °C, preferably wherein the nanofibrillar cellulose in chemically unmodified nanofibrillar cellulose.

11. The bioreactor of any of the claims 1-7, wherein the nanostructured cellulose comprises nanocrystalline cellulose having a number average fibril diameter in the range of 2-40 nm, such as 2-20 nm, and a number average fibril length of 100 nm or more, such as in the range of 100-400 nm.

12. The bioreactor of any of the preceding claims, wherein the nanostructured cellulose has a median pore size in the range of 0.1-10 µm², such as 0.3-2.0 µm².

13. A method for extracting cell-derived products from cultured cells, the method comprising
- providing the bioreactor (10) of any of the preceding claims,
- providing cell culture medium, preferably serum-free, animal origin free, feeder-free and/or xeno-free cell culture medium,
- providing cells,
- incubating the cells in the compartment comprising nanostructured cellulose to form cell-derived products, and optionally mixing the nanostructured cellulose and the cells,
- allowing cell-derived products to diffuse from the cells into the cell culture medium,
- harvesting the cell culture medium comprising the cell-derived products from the bioreactor, preferably via the outlet for extracting the cell culture medium comprising the cell-derived products, to separate the cell-derived products from the incubated cells.

14. The method of claim 13, wherein the method is a continuous method, preferably the method comprising adding new cell culture medium to the bioreactor at an equal rate to the rate which the cell culture medium comprising cell-derived products is harvested from the bioreactor.

15. The method of claim 13 or 14, wherein the cell-derived products comprise extracellular vesicles, such as exosomes, preferably wherein the cells are mammalian cells, such as human cells, for example wherein the cells are primary cells, such as stem cells, for example mesenchymal stem cells, and/or wherein the cells are present as aggregates, such as spheroids.

16. A nanostructured cellulose product for extracting cell-derived products from cultured cells, the product comprising chemically unmodified nanostructured cellulose hydrogel having a median pore size in the range of 0.1-10 µm², such as 0.3-2.0 µm².

17. The nanostructured cellulose product of claim 16, wherein the nanostructured cellulose is nanofibrillar cellulose comprising cellulose fibrils and/or fibril bundles having a number-average diameter in the range of 2-50 nm, such as 2-20 nm, and wherein the nanofibrillar cellulose, when dispersed in water, provides a yield stress in the range of 1-50 Pa, such as in the range of 2-15 Pa, determined by rotational rheometer at a consistency of 0.5% by weight in aqueous medium at 22±1 °C.
